# FASCICULE DE BREVET EUROPEEN

(11) **EP 2 248 515 B1**
(45) Date de publication et mention de la délivrance du brevet: **23.12.2015**
(21) Numéro de dépôt: 10161248.9
(22) Date de dépôt: 28.04.2010
(51) Int. Cl.: A61K 8/49, A61Q 5/06, C07D 471/04

(54) **Utilisation pour la coloration des fibres kératiniques d'un composé de type azomethinique a motif pyrazolopyridine**
Verwendung zur Färbung keratinischen Fasern von einer Azomethinekomponente enthaltend eine pyrazolopyridine Funktion
Use for dying keratinic fibers of a azomethine-type compound having a pyrazolopyridine group

(30) Priorité: 30.04.2009 FR 0952895
(43) Date de publication de la demande: 10.11.2010
(73) Titulaire: L'Oréal, 75008 Paris (FR)
(72) Inventeur: Fadli, Aziz, 77500, Chelles (FR); Blais, Stéphane, 91120, Palaiseau (FR)
(74) Mandataire: Prevel, Estelle Nicole

(56) Documents cités:
- EP-A1- 2 011 787
- EP-A2- 1 634 574
- DE-A1- 3 942 355
- DE-A1- 4 311 460

## Description

La présente invention a pour objet l'utilisation pour la coloration des fibres kératiniques, et en particulier des fibres kératiniques humaines telles que les cheveux, d'un composé de type azométhinique à motif pyrazolopyridine.

Il est connu de teindre les fibres kératiniques avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique, azinique ou triarylméthane.

Habituellement, ces colorants sont appliqués sur les fibres, éventuellement en présence d'un agent oxydant, si l'on souhaite obtenir un effet simultané d'éclaircissement des fibres. Une fois le temps de pose écoulé, les fibres sont rincées, éventuellement lavées et séchées.

Les colorations résultant de l'utilisation de colorants directs sont des colorations souvent chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur relative mauvaise tenue aux lavages ou à la transpiration. Ces colorants directs sont en outre généralement sensibles à la lumière car la résistance du chromophore vis-à-vis des attaques photochimiques est faible, ce qui conduit à un affadissement de la coloration des cheveux dans le temps. La sensibilité de ces colorants à la lumière dépend de leur répartition uniforme ou en agrégats dans et/ou sur la fibre kératinique.

Pour obtenir le même résultat, il est également possible d'utiliser la forme réduite non colorée de ces colorants et de l'appliquer sur les fibres kératiniques en présence d'oxydant pour générer la forme oxydée colorée et colorante. La coloration obtenue peut alors être effacée puis reformée rapidement en passant d'une forme à l'autre.

Ainsi, il est connu de la demande de brevet français FR 2 917 737 d'utiliser des composés de type azométhinique à motif pyrazolinone et leurs formes réduites pour obtenir une coloration des fibres kératiniques qui peut s'effacer puis se reformer facilement.

Le but de la présente invention est de fournir de nouveaux colorants directs permettant de colorer les fibres kératiniques de manière réversible tout en conduisant à de bonnes propriétés tinctoriales.

En particulier, l'un des buts de la présente invention est de fournir des colorants directs qui permettent d'obtenir une coloration aux nuances variées, puissante, chromatique, esthétique, peu sélective et résistant bien aux diverses agressions que peuvent subir les cheveux tels que les shampooings, la lumière, la sueur et les déformations permanentes, et qui peut s'effacer facilement.

Ce but est atteint avec la présente invention qui a pour objet l'utilisation pour la coloration des fibres kératiniques d'au moins un composé choisi parmi les composés de type leuco de formule (I), les colorants de type azométhinique à motif pyrazolopyridine de formule (II) correspondant aux composés de formule (I), leurs formes mésomères, isomères et tautomères, ainsi que leurs sels d'addition avec un acide et leurs solvates : 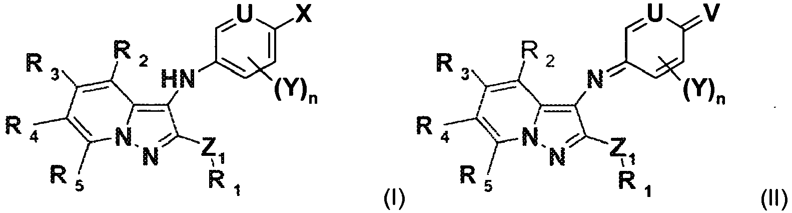 dans lesquelles :
- Z₁ représente un atome d'oxygène, un groupement NR₆, lorsque Z₁ représente NR₆ alors R₁ et R₆ peuvent former ensemble avec l'atome d'azote auquel ils sont rattachés un hétérocycle saturé, insaturé ou aromatique de 5 à 8 chaînons, éventuellement substitué,
   Z₁ peut aussi représenter un radical divalent S, SO, SO₂ lorsque R₁ est un radical méthyle,
- R₁ et R₆ représentent indépendamment :
   - un atome d'hydrogène,
   - un radical alkyle en C₁-C₁₀, éventuellement substitué, le substituant pouvant éventuellement être un (hétéro)cycle saturé, insaturé ou aromatique de 5 à 8 chaînons, éventuellement substitué,
   - un (hétéro)cycle saturé, insaturé ou aromatique de 5 à 8 chaînons, éventuellement substitué,
- R₂, R₃, R₄, R₅, indépendamment, représentent :
   - un atome d'hydrogène,
   - un radical alkyle en C₁-C₄ éventuellement substitué,
   - un groupement choisi parmi NH₂, NHR₁₀, NR₁₁R₁₂, OH, OR₉, avec R₉ et R₁₀ représentant un alkyle en C₁-C₆ linéaire ou ramifié, éventuellement substitué, R₁₁ et R₁₂ identiques ou différents représentant un alkyle en C₁-C₆ linéaire ou ramifié, éventuellement substitué, R₁₁ et R₁₂ pouvant former ensemble avec l'atome d'azote auquel ils sont rattachés, un hétérocycle saturé, insaturé, ou aromatique, de 5 à 8 chaînons renfermant éventuellement un ou plusieurs autres hétéroatomes ou groupements choisis parmi N, O, S, SO₂, CO, l'hétérocycle étant éventuellement substitué,
- R₂, R₃, R₄, R₅, peuvent former deux à deux avec des radicaux adjacents un (hétéro)cycle saturé ou insaturé, éventuellement substitué,
- n est un entier compris entre 0 et 3 ;
- U représente CR ou N ;
- R représente
   - un atome d'hydrogène,
   - un radical alkyle en C₁-C₄ éventuellement substitué par un radical hydroxyle ;
   - un radical alcoxy en C₁-C₄ éventuellement substitué par un radical hydroxyle ;
   - un radical (di)alkyl(C₁-C₄)amino dont la partie alkyle est éventuellement substituée par un radical hydroxyle ;
- X représente :
   - un radical hydroxyle;
   - un groupement NR'₁R"₁ avec R'₁ et R"₁ choisis indépendamment parmi un atome d'hydrogène ; un radical alkyle en C₁-C₆ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy ;
   lorsque R'₁ et R"₁ sont différents de l'hydrogène, R'₁ et R"₁ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, carboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
   lorsque X représente un groupement NHR'₁ et que U représente un groupement CR dans lequel R désigne un radical alcoxy, alors X et U peuvent former un cycle à 6 chainons de type morpholine, éventuellement substitué par un ou plusieurs groupements alkyles en C₁-C₄ ;
- V représente
   o un atome d'oxygène ;
   o un groupement NR'₁ avec R'₁ choisi parmi un atome d'hydrogène ; un radical alkyle en C₁-C₆ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy ;
   lorsque V représente un groupement NR'₁ et que U représente un groupement CR dans lequel R désigne un radical alcoxy, alors V et U peuvent former un cycle à 6 chainons de type morpholine, éventuellement substitué par un ou plusieurs groupements alkyles C₁-C₄ ;
- Y, identiques ou différents, représentent :
   - un radical hydroxy ;
   - un radical alkyle en C₁-C₄ ;
   - un radical hydroxyalkyle C₁-C₄ ;
   - un atome d'halogène tel qu'un atome de chlore, d'iode, de fluor ou de brome ;
   - un atome d'oxygène substitué par un radical choisi parmi un radical alkyle en C₁-C₄, un radical aryle et un radical hétéroaryle, ceux-ci pouvant être substitués par un ou plusieurs radicaux hydroxy ;
   - un groupement NR'₂R'₃ ;
      R'₂ et R'₃, identiques ou différents, peuvent être choisis parmi
         - un atome d'hydrogène ;
         - un radical alkylcarbonyle en C₁-C₄ éventuellement substitué par un groupement ammonium quaternaire tel que par exemple un trialkylammonium ou par un hétérocycle azoté cationique ou non comme par exemple un groupement imidazole, un groupement thiazole, un groupement pyridine, un groupement pipéridine, un groupement pyrrolidine, un groupement pyrimidine, un groupement pyrazine, un groupement imidazolium, un groupement pyridinium, un groupement thiazolium, un groupement pyrrolidinium, un groupement piperidinium, un groupement pyrimidinium, ces hétérocycles azotés étant eux-mêmes éventuellement substitués par un ou plusieurs radicaux alkyle en C₁-C₄;
   - un radical aminocarbonyle ;
   - un radical alkyle en C₁-C₆ éventuellement substitué par un ou plusieurs radicaux choisis parmi un radical hydroxy, (C₁-C₂)alcoxy, amino, (di)alkyl(C₁-C₂)amino ;
   - un radical phényle éventuellement substitué par un ou plusieurs radicaux choisis parmi un radical hydroxy, amino, (C₁-C₂)alcoxy ;
      R'₂ et R'₃ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
      deux radicaux Y portés par deux atomes de carbone adjacents peuvent former ensemble avec les atomes de carbone auxquels ils sont rattachés un groupement cyclique ou hétérocyclique, saturé ou insaturé, aromatique ou non aromatique, comportant de 5 à 6 chaînons, par exemple un cycle benzène, pyrrole, pyrrolidine, pyrazole, furanne, pyrrolidine, morpholine ou imidazole, éventuellement substitué par un ou plusieurs radicaux alkyle en C₁-C₄.

La présente invention a aussi pour objet les nouveaux composés choisis parmi les composés de type leuco de formule (I), les colorants de type azométhinique à motif pyrazolopyridine de formule (II) correspondant aux composés de formule (I), leurs formes mésomères, isomères et tautomères, leurs sels d'addition avec un acide et leurs solvates.

La présente invention a également pour objet une composition pour la coloration des fibres kératiniques comprenant, dans un milieu approprié pour la teinture, au moins un composé choisi parmi les composés de type leuco de formule (I), les colorants de type azométhinique à motif pyrazolopyridine de formule (II) correspondant aux composés de formule (I), leurs formes mésomères, isomères et tautomères, leurs sels d'addition avec un acide et leurs solvates.

La présente invention a également pour objet un procédé pour la coloration des fibres kératiniques mettant en oeuvre des composés choisis parmi les composés de type leuco de formule (I), les colorants de type azométhinique à motif pyrazolopyridine de formule (II) correspondant aux composés de formule (I), leurs formes mésomères, isomères et tautomères, leurs sels d'addition avec un acide et leurs solvates.

La présente invention a aussi pour objet un dispositif à plusieurs compartiments pour la mise en oeuvre du procédé conforme à l'invention.

A moins d'une indication différente, les bornes des gammes de valeurs qui sont données dans le cadre de l'invention sont incluses dans ces gammes.

Dans le cadre de l'invention, sauf indication contraire, on entend par radical alkyle, les radicaux alkyles linéaires ou ramifiés, pouvant être substitués ou non substitués. Sauf liste explicite, ils peuvent être substitués par n'importe quel substituant classique dans le domaine de la coloration et qui ne changent pas les propriétés de colorant des composés de formule (I) et / ou (II).

Un radical alcoxy est un radical alkyl-O-, le radical alkyle étant tel que défini précédemment.

Un radical (di)alkylamino est un radical amino qui peut être substitué par un ou deux radicaux alkyle.

Un radical (di)alkylcarboxamido est un radical carboxamido qui peut être substitué par un ou deux radicaux alkyle.

De même, lorsque les radicaux (hétéro)cycliques définis pour la formule (I) et / ou (II) sont substitués, ils peuvent être substitués par n'importe quel radical classique dans le domaine de la coloration qui ne change pas les propriétés de colorant des composés de formule (I) et / ou (II). A titre d'exemples de substituants des cycles ou hétérocycles, on peut citer les radicaux alkyles, les radicaux alkyles substitués, les radicaux hydroxy, alcoxy, amino, alkylamino, dialkylamino, thio, alkylthio, carboxy, alkylcarbonyle, sulfonyle, amido, etc.

L'hétérocycle azoté formé par R₁ et R₆ peut contenir un ou plusieurs autres hétéroatomes notamment un hétéroatome choisi parmi N, O, S, SO, SO₂, -CO- et leurs combinaisons. Il peut de plus être substitué ou non, notamment comme décrit ci-dessus.

Dans les formules (I) et (II) ci-dessus, lorsque R₁ et / ou R₆ représentent un radical alkyle substitué, alors les substituants sont notamment choisis parmi les halogènes, les radicaux -OH, -OR₉, -NH₂, -NHR₁₀, -NR₁₁R₁₂, -COR₁₃, -O-CO-R₁₃, -CO-OR₁₄, -NR₁₅-CO-R₁₆, -CO-NR₁₅R₁₆, -SO₃H, les radicaux cycliques saturés ou insaturés contenant éventuellement un hétéroatome choisi parmi N, S, O, le cycle pouvant être lui-même substitué, dans lesquelles R₉, R₁₀ ,R₁₁ et R₁₂ identiques ou différents sont tels que définis précédemment ; R₁₃, R₁₄ R₁₅ et R₁₆, identiques ou différents, représentent un hydrogène ou un radical alkyle en C₁-C₆. Dans les formules (I) et (II) ci-dessus, lorsque R₁ et / ou R₆ représentent un radical alkyle substitué, alors les substituants sont choisis également parmi les radicaux -OSO₂R, R représentant une radical alkyle linéaire ou ramifié en C₁-C₄ ou un radical aromatique éventuellement substitué. A titre d'exemple, on peut citer les radicaux -OH, -OR₉, -NH₂, -NHR₁₀, -NR₁₁R₁₂, -COR₁₃, les radicaux cycliques de type imidazole, pipérazine, pyrrolidine, pyridine, pipéridine, morpholine, pyrimidine.

Selon un mode de réalisation particulier de l'invention, les composés de formule (I) ou (II) ci-dessus sont tels que Z₁ représente un atome d'oxygène, un radical NR₆ ou un radical NR₆ formant avec R₁ un hétérocycle.

Le radical R₆ peut être choisi parmi un atome d'hydrogène, un radical alkyle en C₁-C₆ ou un radical alkyle en C₁-C₆ substitué par un radical hydroxy, un radical amino, un radical alkyl(C₁-C₄)amino, un radical dialkyl(C₁-C₄)amino, un radical alkyle en C₁-C₆ substitué par un hétérocycle azoté par exemple pipérazinyle, imidazolyle, pyrrolidinyle, pyridinyle, morpholinyle, pipéridinyle.

Selon ce mode de réalisation, Z₁ représente de préférence un atome d'oxygène ou un radical NH.

Selon l'invention, le radical R₁ peut être choisi parmi un radical alkyle en C₁-C₆, un radical alkyle en C₁-C₆ substitué par un hydroxy, un radical alkyle en C₁-C₆ substitué par un amino ou (di)alkyl(C₁-C₄)amino, un radical alkyle en C₁-C₆ substitué par un hétérocycle azoté par exemple pipérazinyle, imidazolyle, pyrrolidinyle, pyridinyle, morpholinyle, pipéridinyle. Par exemple, R₁ est choisi parmi un radical alkyle en C₁-C₆ substitué par un amino ou un alkyl(C₁-C₄)amino.

De préférence, le radical R₁ représente un radical alkyle en C₁-C₆, un radical alkyle en C₁-C₆ substitué par un hydroxy, un radical alkyle en C₁-C₆ substitué par un amino ou (di)alkyl(C₁-C₄)amino, un radical alkyle en C₁-C₆ substitué par un hétérocycle azoté choisi parmi imidazolyle, pyrrolidinyle, pipéridinyle.

Selon le mode de réalisation particulier dans lequel R₁ et R₆ forment ensemble un hétérocycle avec l'atome d'azote auquel ils sont rattachés, alors l'hétérocycle peut être choisi parmi les imidazoles, les pipérazines, les pyrrolidines, les diazépanes, ces hétérocycles pouvant être substitués ou non.

Lorsque R₂, R₃, R₄ et R₅ représentent un radical alkyle substitué, alors ce radical alkyle peut être notamment substitué par un groupement choisi parmi OH, OR₉, NH₂, NHR₁₀, NR₁₁R₁₂ ou SR₉ dans lesquelles R₉, R₁₀, R₁₁ et R₁₂ identiques ou différents sont tels que définis précédemment.

A titre d'exemple, on peut citer les radicaux méthyle, éthyle, hydroxyéthyle, aminoéthyle, propyle, butyle.

Selon une variante, R₂, R₃, R₄ et R₅ représentent indépendamment un atome d'hydrogène ou un radical alkyle en C₁-C₄ tel que méthyle, éthyle, propyle, isopropyle, butyle, tertiobutyle, ou R₄ et R₅ forment ensemble un cycle de 5 à 8 chaînons. Selon un mode de réalisation particulier, R₄ et R₅ forment ensemble un cycle saturé ou insaturé de 5 à 8 chaînons, notamment de 5 à 6 chaînons, par exemple un cyclopentane ou cyclohexane, éventuellement substitué.

De préférence, R₂, R₃, R₄ et R₅ représentent indépendamment un atome d'hydrogène ou un radical alkyle en C₁-C₄ tel que méthyle, éthyle, propyle, isopropyle, butyle, tertiobutyle.

Selon un mode de réalisation particulier de l'invention, U représente CR ou N, et R représente un atome d'hydrogène, un radical alkyle en C₁-C₄, un radical alcoxy en C₁-C₄ éventuellement substitué par un radical hydroxyle, un radical (di)alkyl(C₁-C₄)amino dont la partie alkyle est éventuellement substituée par un radical hydroxyle.

De préférence, U représente CR ou N, et R représente un atome d'hydrogène, un radical méthyle, un radical méthoxy, un radical 2-hydroxyéthoxy, un radical méthylamino, un radical diméthylamino ou hydroxyéthylamino ou un radical dihydroxyéthylamino ou un radical méthyl(hydroxyéthyl)amino.

De façon encore plus préférée, U représente CR ou N, et R représente un atome d'hydrogène, un radical méthyle, un radical 2-hydroxyéthoxy.

Selon un mode de réalisation particulier de l'invention, X représente un radical hydroxyle ; un groupement NR'₁R "₁ avec R'₁ et R"₁ choisis indépendamment parmi un atome d'hydrogène ; un radical alkyle en C₁-C₆ éventuellement substitué par un ou plusieurs radicaux hydroxyle. De préférence, X représente un radical hydroxyle; un groupement NR'₁R"₁ avec R'₁ et R"₁ choisis indépendamment parmi un atome d'hydrogène ; un radical méthyle ; un radical 2-hydroxyéthyle.

Selon un autre mode de réalisation, lorsque R'1 et R"1 forment un hétérocycle alors cet hétérocycle peut être choisi parmi les hétérocycles pyrrolidine, pipéridine, homopipéridine, pipérazine, homopipérazine, morpholine ; les dits cycles pouvant être substitués par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle. A titre d'exemple, ces hétérocyles sont choisis parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, la 3-hydroxypipéridine, la 4-hydroxypipéridine, la 3-hydroxyméthylpipéridine, l'homopipéridine, la 2-carboxyhomopipéridine, la 2-carboxamidohomopipéridine, l'homopipérazine, le N-méthyl-homopipérazine, le N-(2-hydroxyéthyl)-homopipérazine, la morpholine.

Plus préférentiellement, ces hétérocyles sont choisis parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, la 4-hydroxypipéridine, l'homopipéridine, l'homopipérazine, la N-méthyl homopipérazine, la N-(2-hydroxyéthyl)-homopipérazine, la morpholine.

Conformément à un mode de réalisation encore plus préféré de l'invention, les radicaux R₁ et R"₁ forment ensemble avec l'atome d'azote auquel ils sont rattachés un cycle à 5 chaînons tel que la pyrrolidine, la 3-hydroxypyrrolidine, la 3-aminopyrrolidine, la 3-diméthylamino-pyrrolidine.

Selon un mode particulier de l'invention, V représente :
- un atome d'oxygène ;
- un groupement NR'₁ dans lequel R'₁ représente un atome d'hydrogène ou un radical alkyle en C₁-C₆ éventuellement substitué par un ou plusieurs radicaux hydroxyle.

De préférence, V représente un atome d'oxygène ou un groupement NH.

Selon un autre mode de réalisation particulier de l'invention, X et U, ou respectivement V et U, forment un cycle à 6 chainons de type morpholine, éventuellement substitué par un ou plusieurs groupements alkyles en C₁-C₄, de préférence non substitué.

Selon un autre mode de réalisation particulier de l'invention, Y, identiques ou différents, représentent un radical hydroxy ; un radical alkyle en C₁-C₄ ; un atome d'halogène ; un atome d'oxygène substitué par un radical alkyle en C₁-C₄ pouvant être substitué par un ou plusieurs radicaux hydroxy ; un groupement NR'₂R'₃ ;
R'₂ et R'₃, identiques ou différents, peuvent être choisis parmi un atome d'hydrogène ; un radical alkylcarbonyle en C₁-C₄ ; un radical aminocarbonyle ; un radical alkyle en C₁-C₆ éventuellement substitué par un ou plusieurs radicaux hydroxy ;
R'₂ et R'₃ peuvent former ensemble avec l'atome d'azote auquel ils sont rattachés un hétérocycle saturé comportant de 5 à 7 chaînons.

Dans un mode de réalisation particulier de l'invention, ces hétérocycles sont choisis parmi les hétérocycles pyrrolidine, pipéridine, homopipéridine, pipérazine, homopipérazine, morpholine ; les dits cycles pouvant être substitués par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle.

Deux radicaux Y portés par deux atomes de carbone adjacents peuvent former ensemble avec les atomes de carbone auxquels ils sont rattachés un groupement cyclique ou hétérocyclique, saturé ou insaturé, aromatique ou non aromatique, comportant 5 ou 6 chaînons.

De préférence, Y, identiques ou différents, représentent un radical hydroxy ; un radical méthyle ; un atome de chlore ; un radical 2-hydroxy éthoxy ; un radical amino ; un radical (2-hydroxyéthyl)amino.

Par sels d'addition, on entend les sels d'acides organiques ou minéraux physiologiquement acceptables des composés de formule (I) et / ou (II).

Les composés de type leuco de formule (I), les colorants de type azométhinique à motif pyrazolopyridine de formule (II) correspondant aux composés de formule (I), peuvent être éventuellement salifiés par des acides minéraux forts tels que par exemple HCl, HBr, Hl, H₂SO₄, H₃PO₄, ou des acides organiques tels que, par exemple, l'acide acétique, lactique, tartrique, citrique ou succinique, benzènesulfonique, para-toluènesulfonique, formique, méthanesulfonique.

Dans le cadre de l'invention, on entend par dérivé de formule (I) et / ou (II) toutes formes mésomères, tautomères ou isomères.

Ils peuvent aussi être sous forme de solvates par exemple un hydrate ou un solvate d'alcool linéaire ou ramifié tel que l'éthanol ou l'isopropanol.

La présente invention permet en particulier d'obtenir rapidement des colorations chromatiques et résistantes aux diverses agressions que peuvent subir les cheveux, notamment aux shampooings et à la lumière, pouvant être effacées puis reformées tout aussi rapidement.

Les composés de type leuco de formule (I), sont incolores ou faiblement colorés et les dérivés azométhiniques à motif pyrazolopyridine correspondants de formule (II), sont des espèces colorées et colorantes. Il est possible de modifier la structure des composés de formule (I) pour obtenir les composés de formule (II) par ajout d'un agent oxydant, et inversement il est possible de modifier la structure des composés de formule (II) pour obtenir les composés de formule (I) par ajout d'un agent réducteur. Cette modification de structure peut être facilitée par modification du pH et / ou de la température. La formation des composés de formule (I) est ainsi favorisée par un pH acide et / ou une diminution dela température, la formation des composés de formule (II) est favorisée par un pH basique et / ou une élévation de la température. Un tel comportement permet notamment de modifier facilement la coloration des fibres kératiniques.

A titre d'exemples de colorants de type azométhinique à motif pyrazolopyridine de formule (I) et / ou (II), on peut citer les composés présentés ci-dessous :

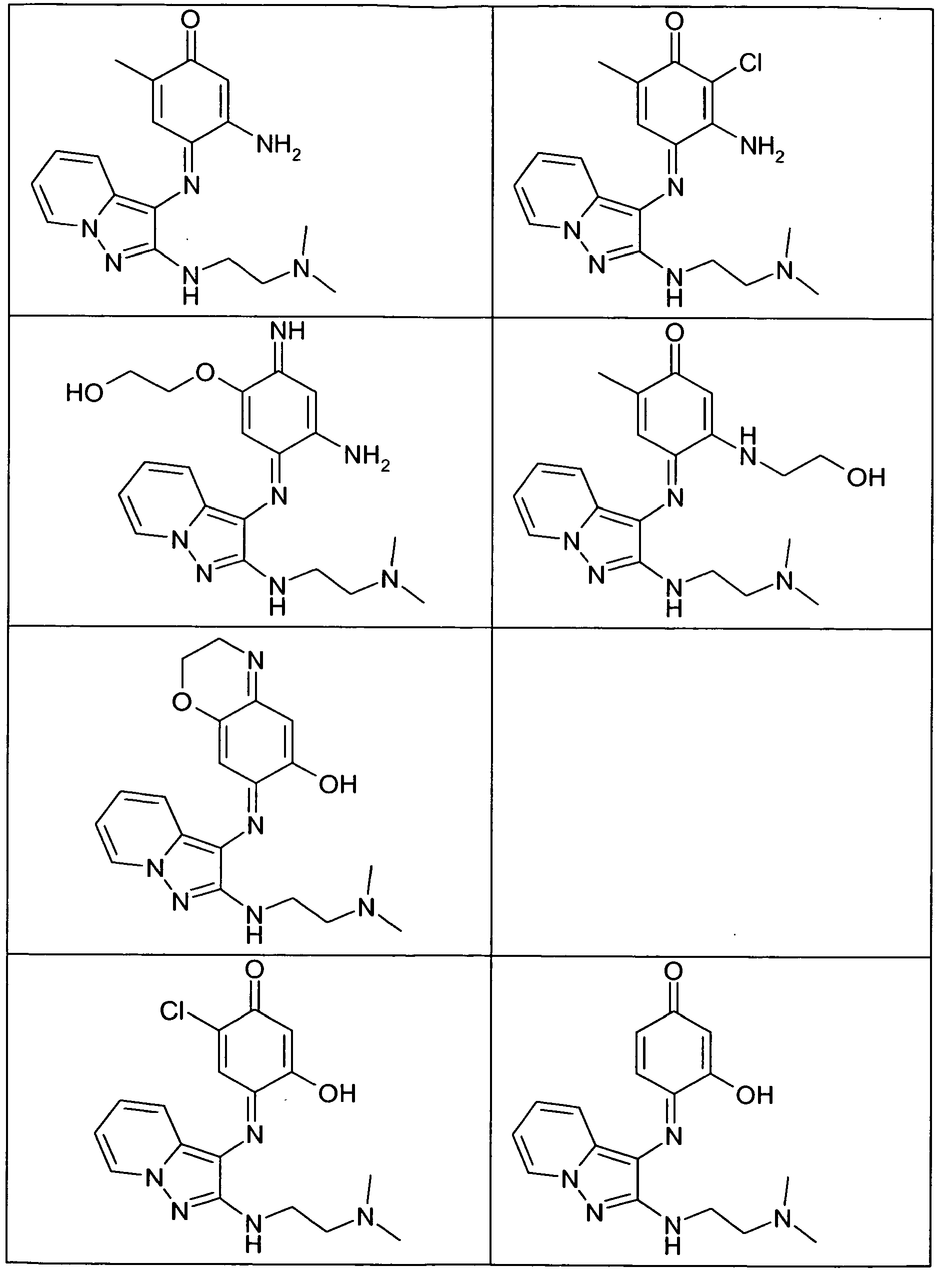

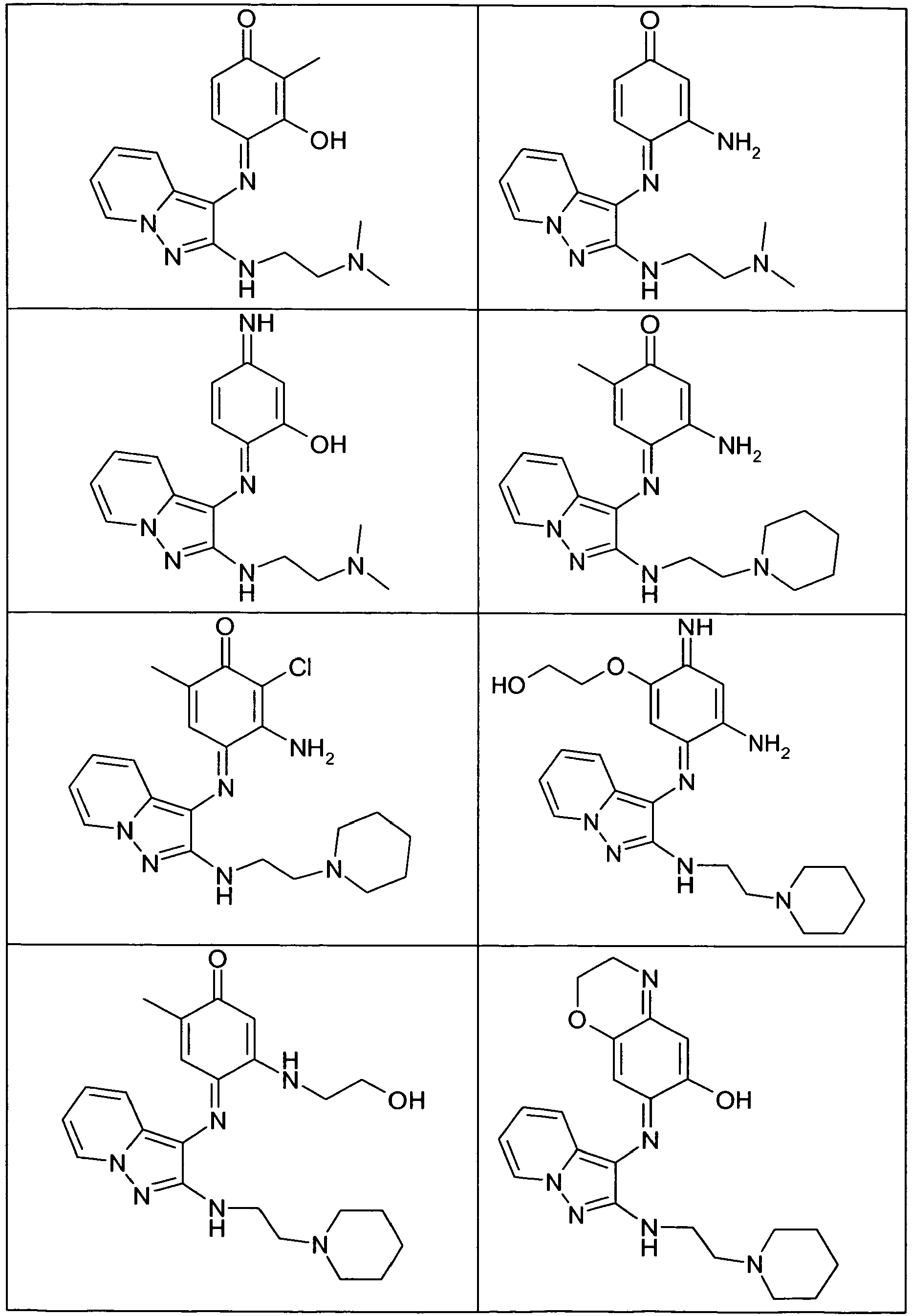

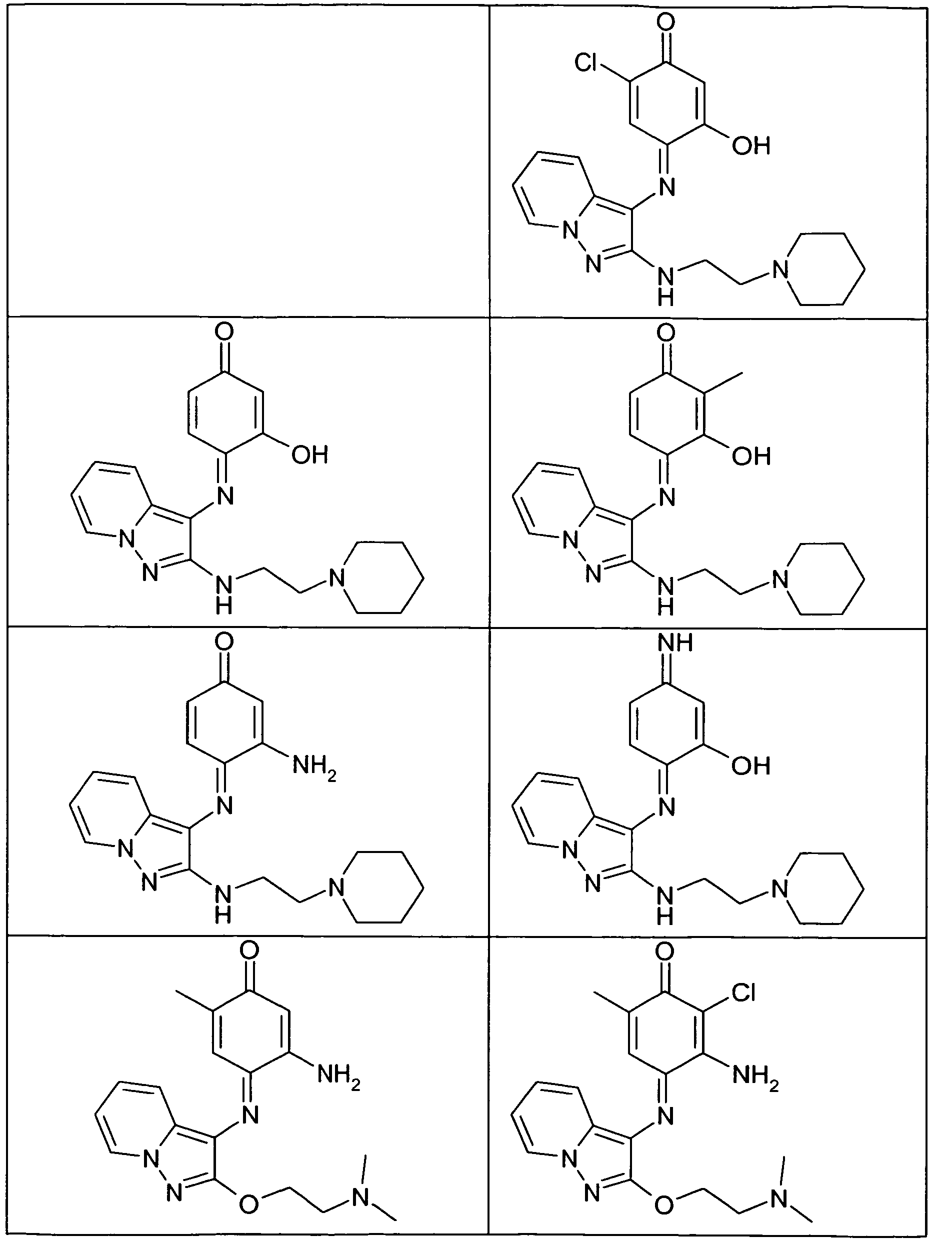

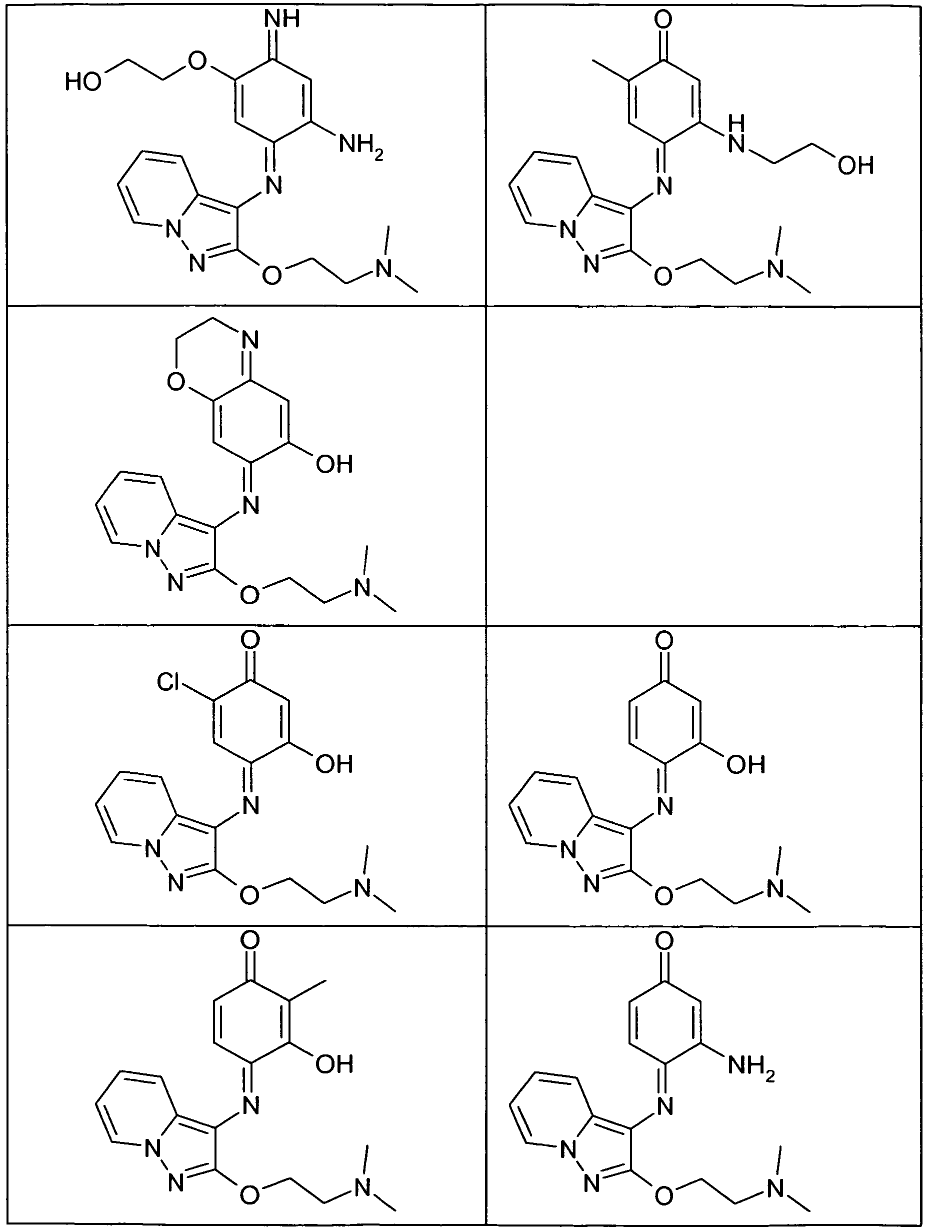

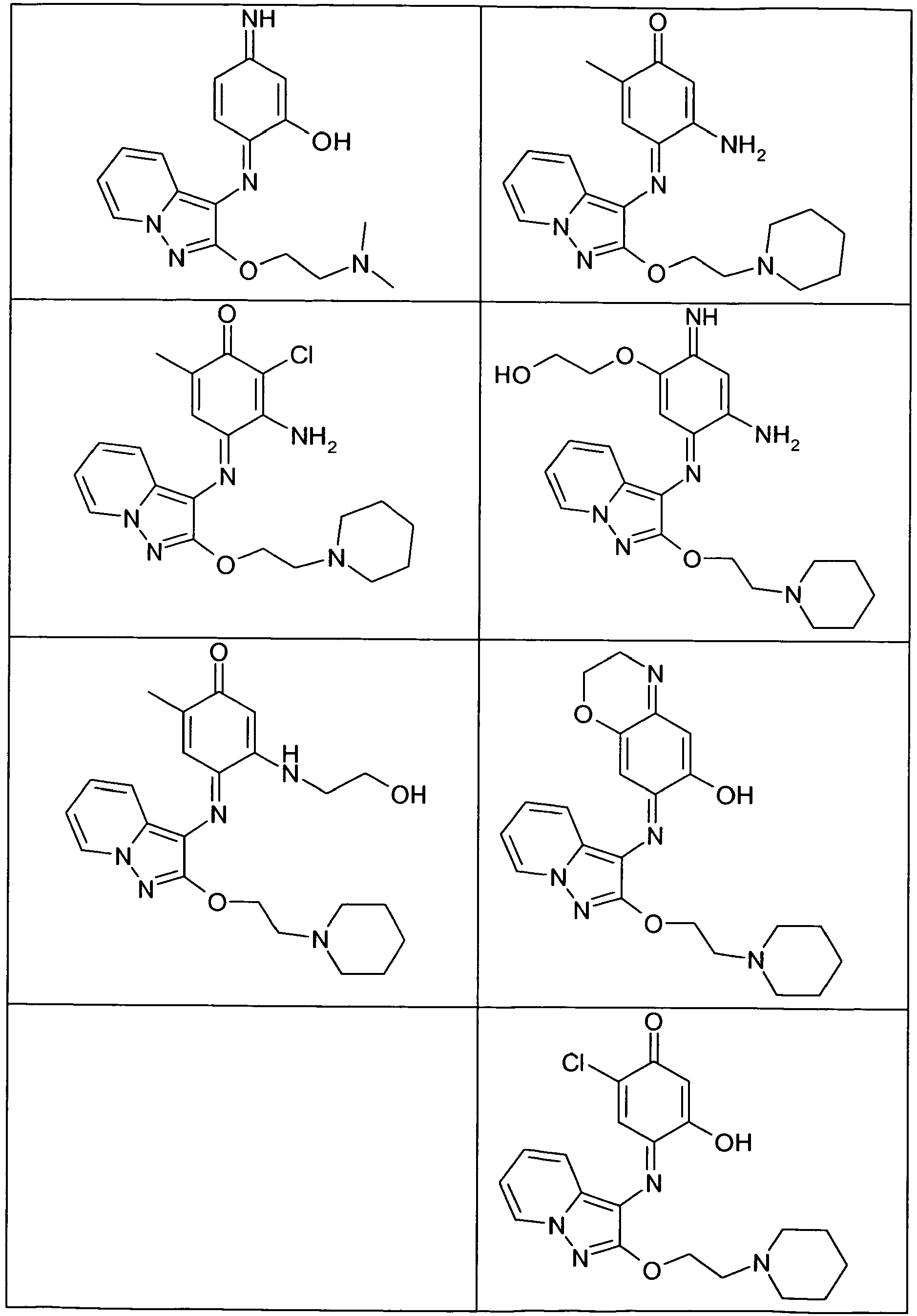

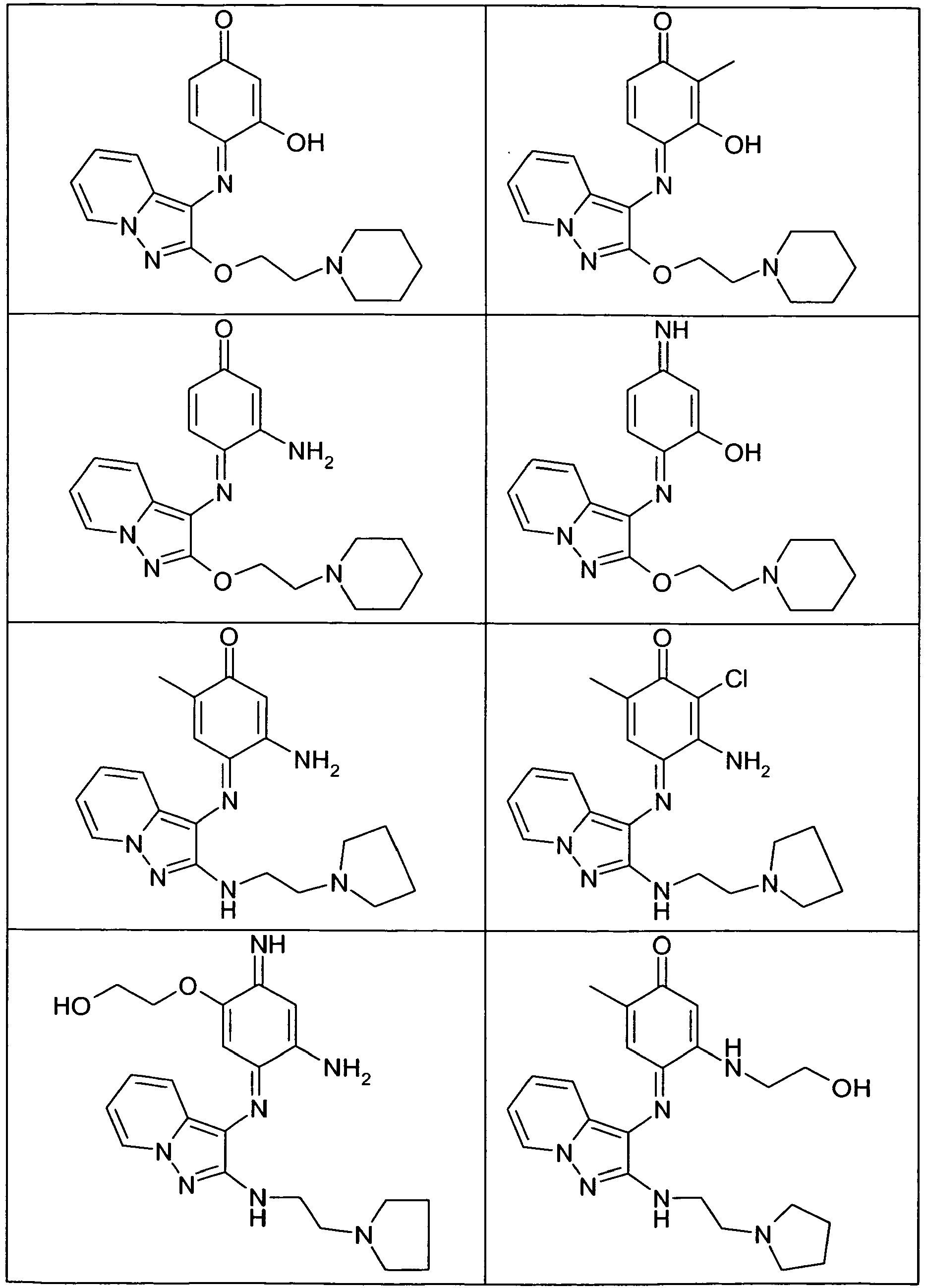

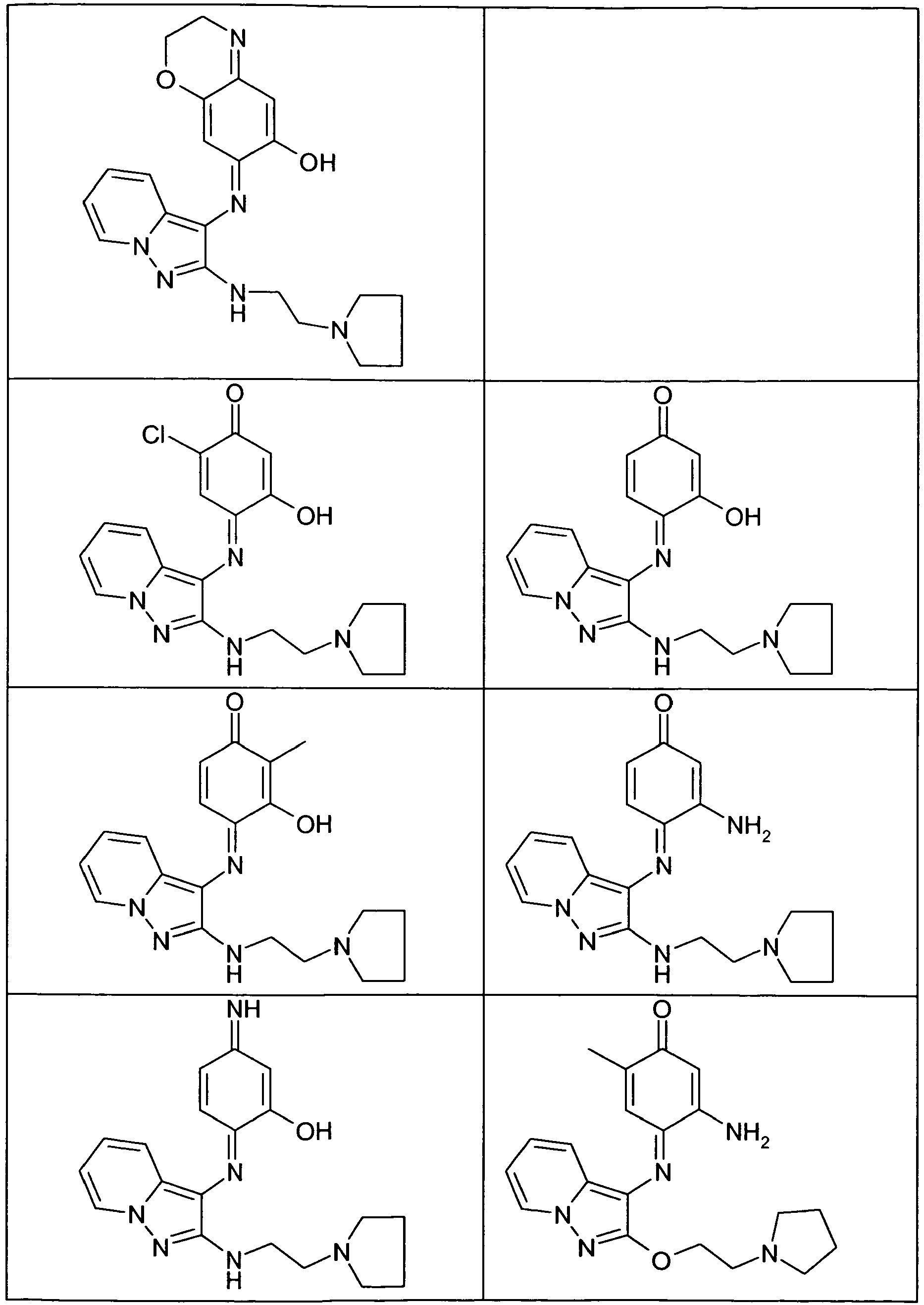

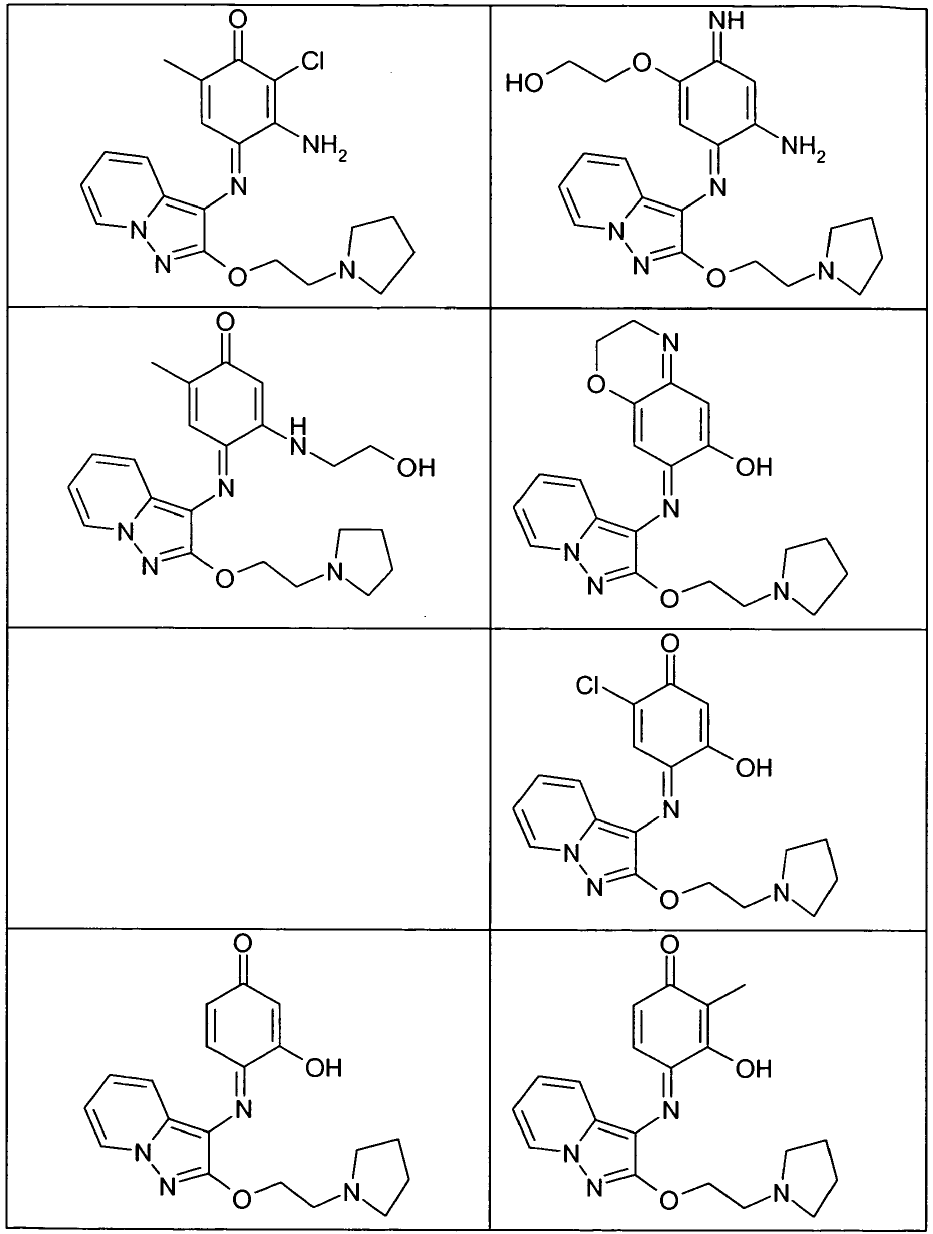

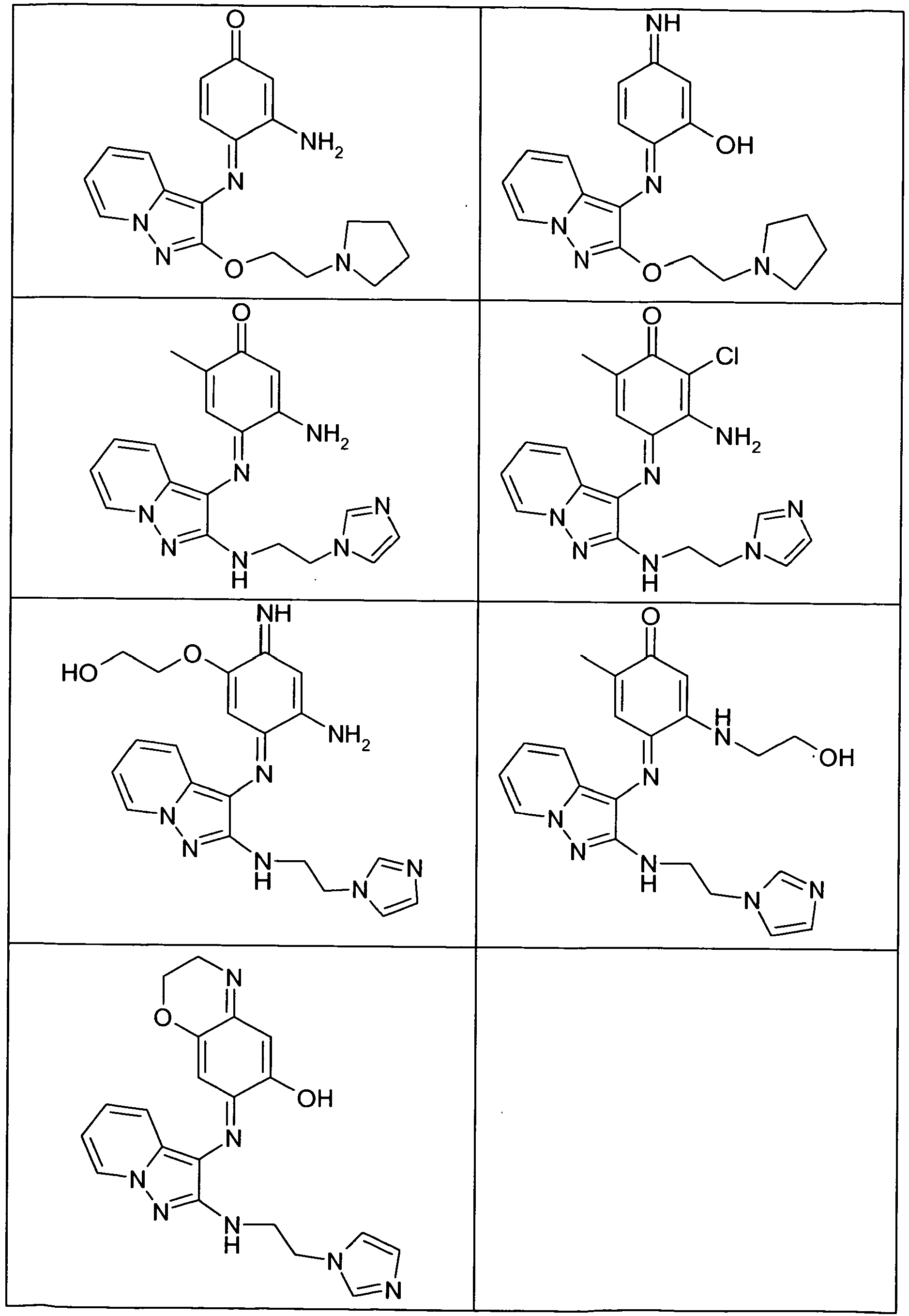

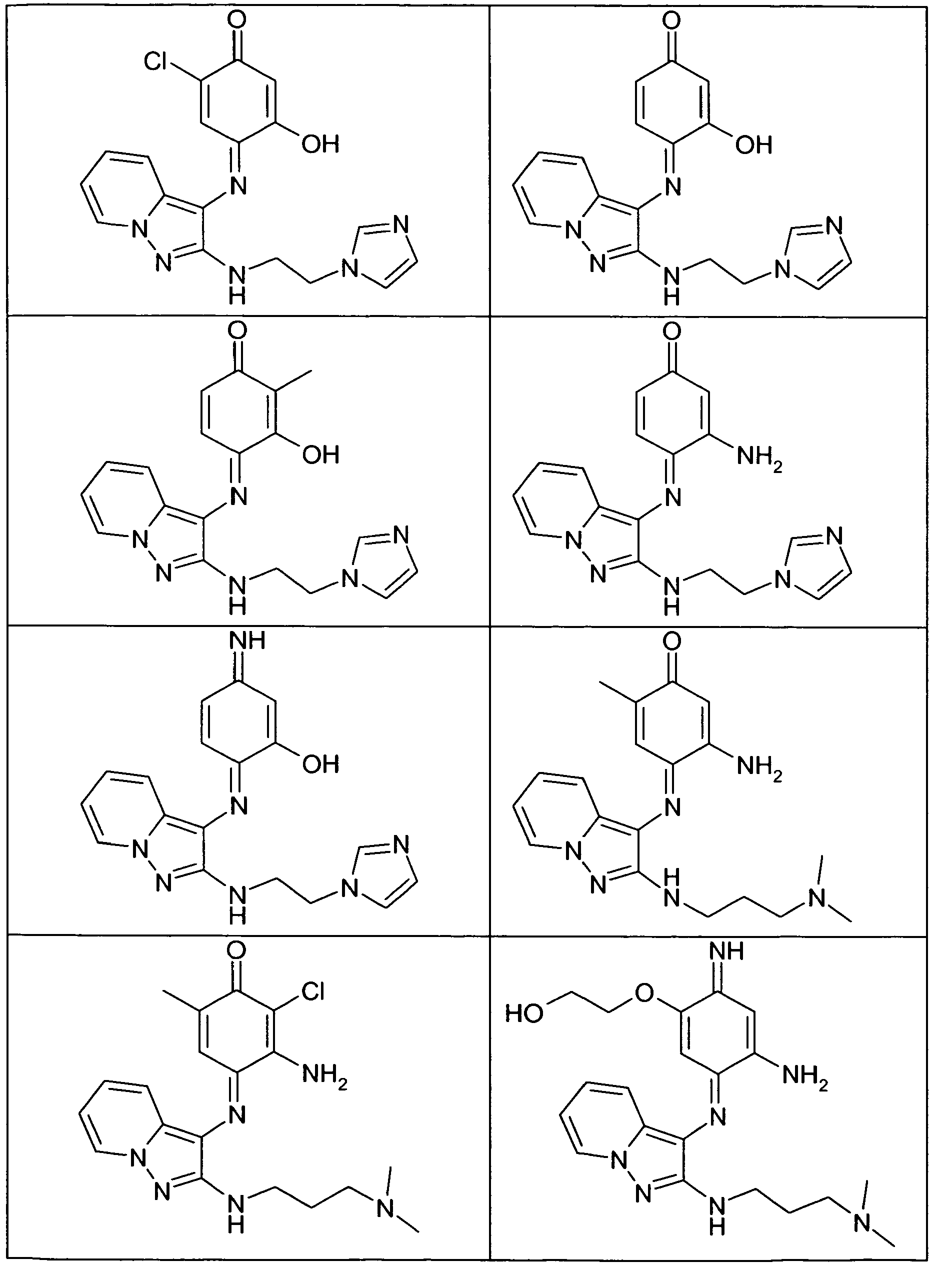

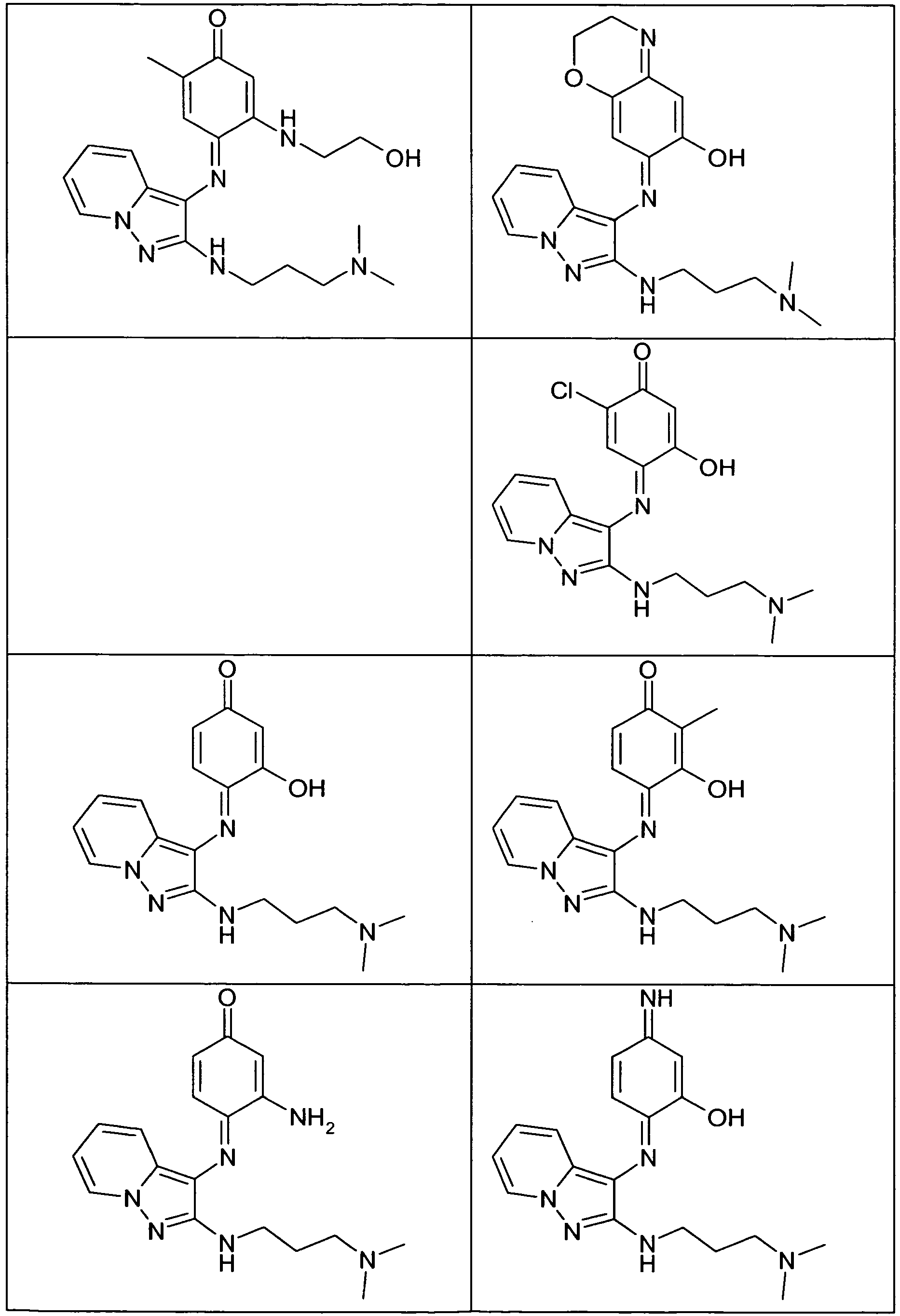

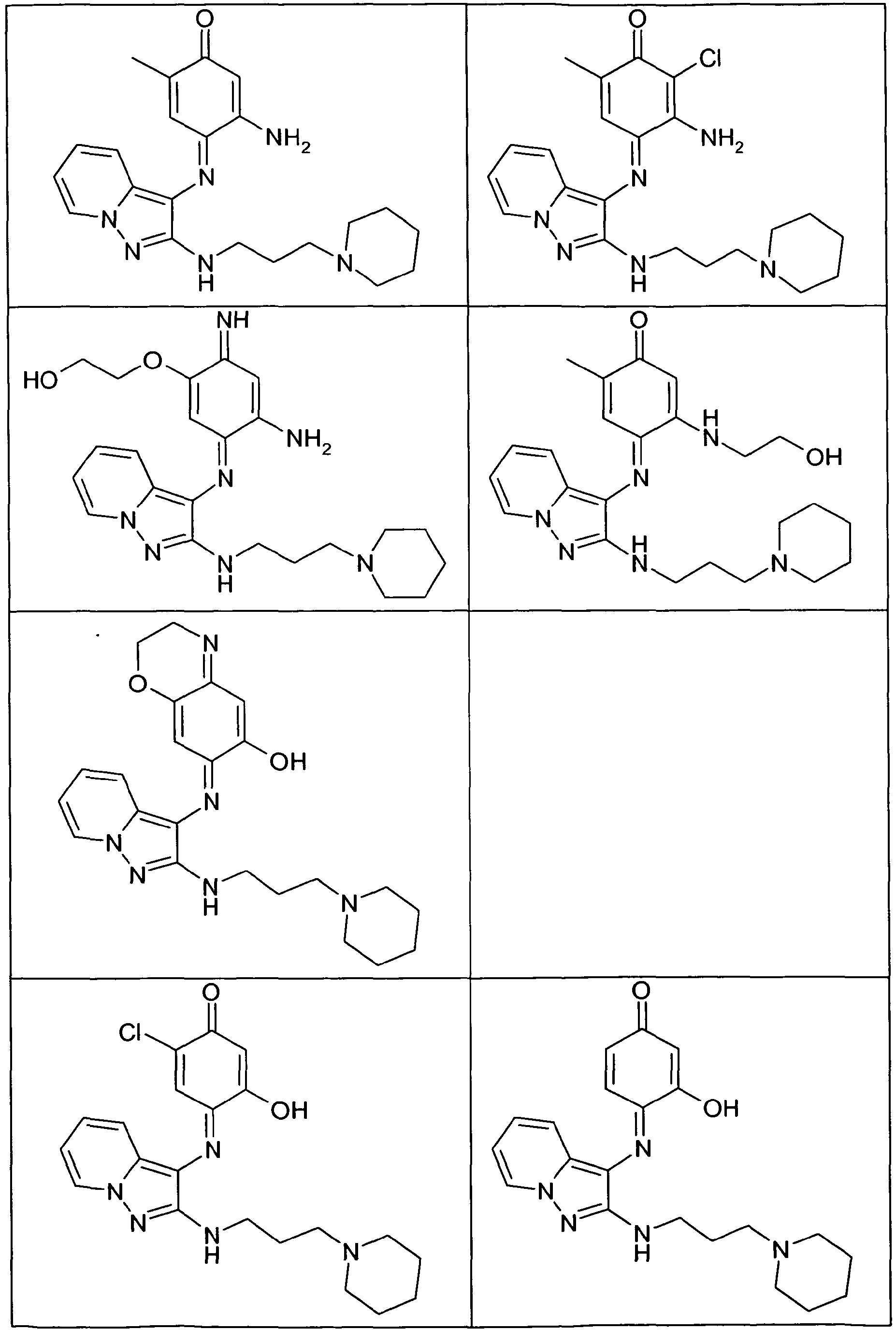

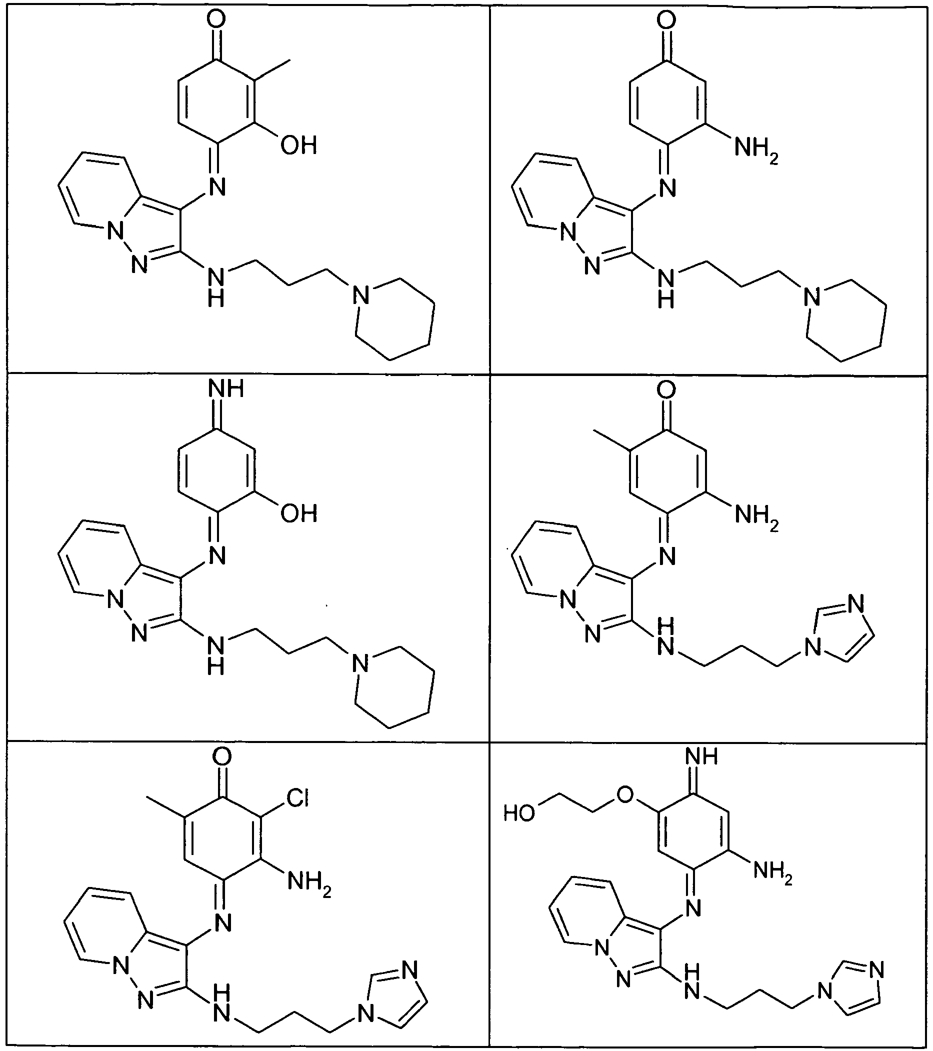

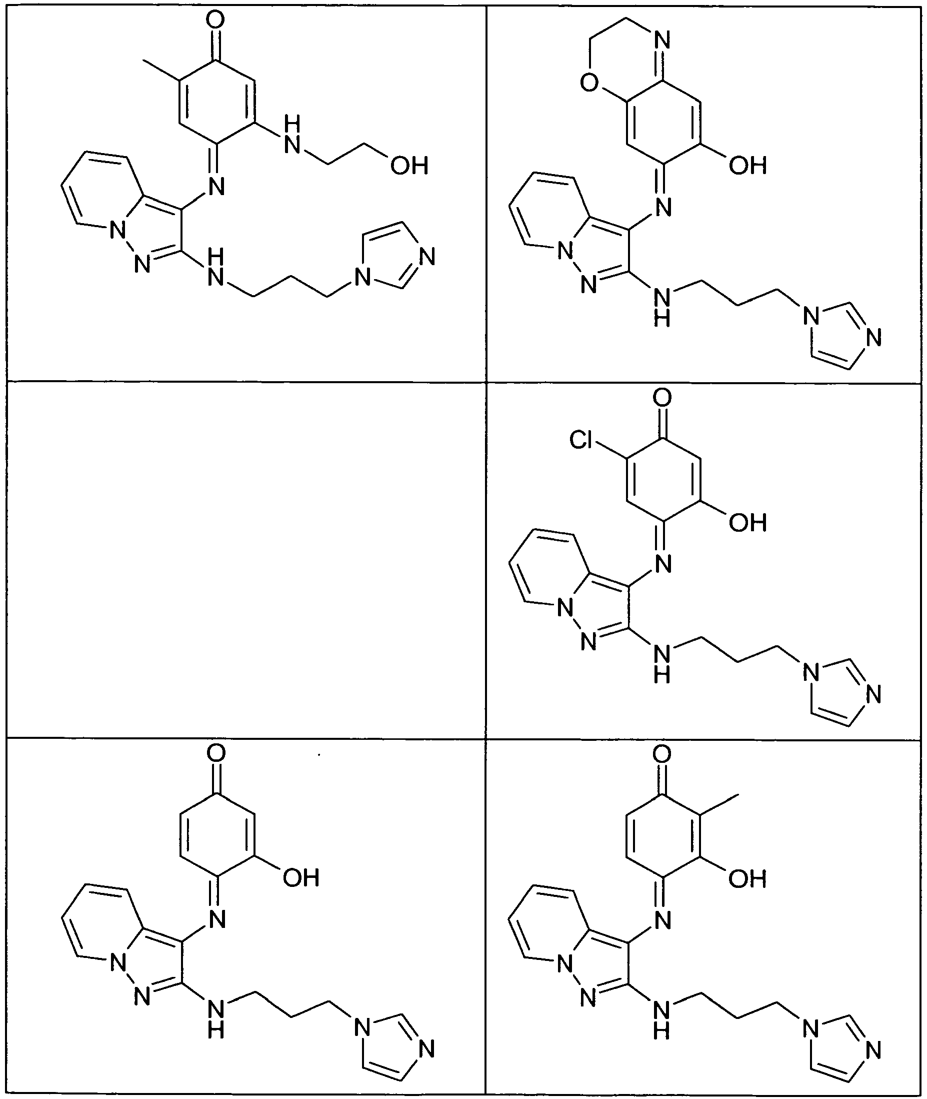

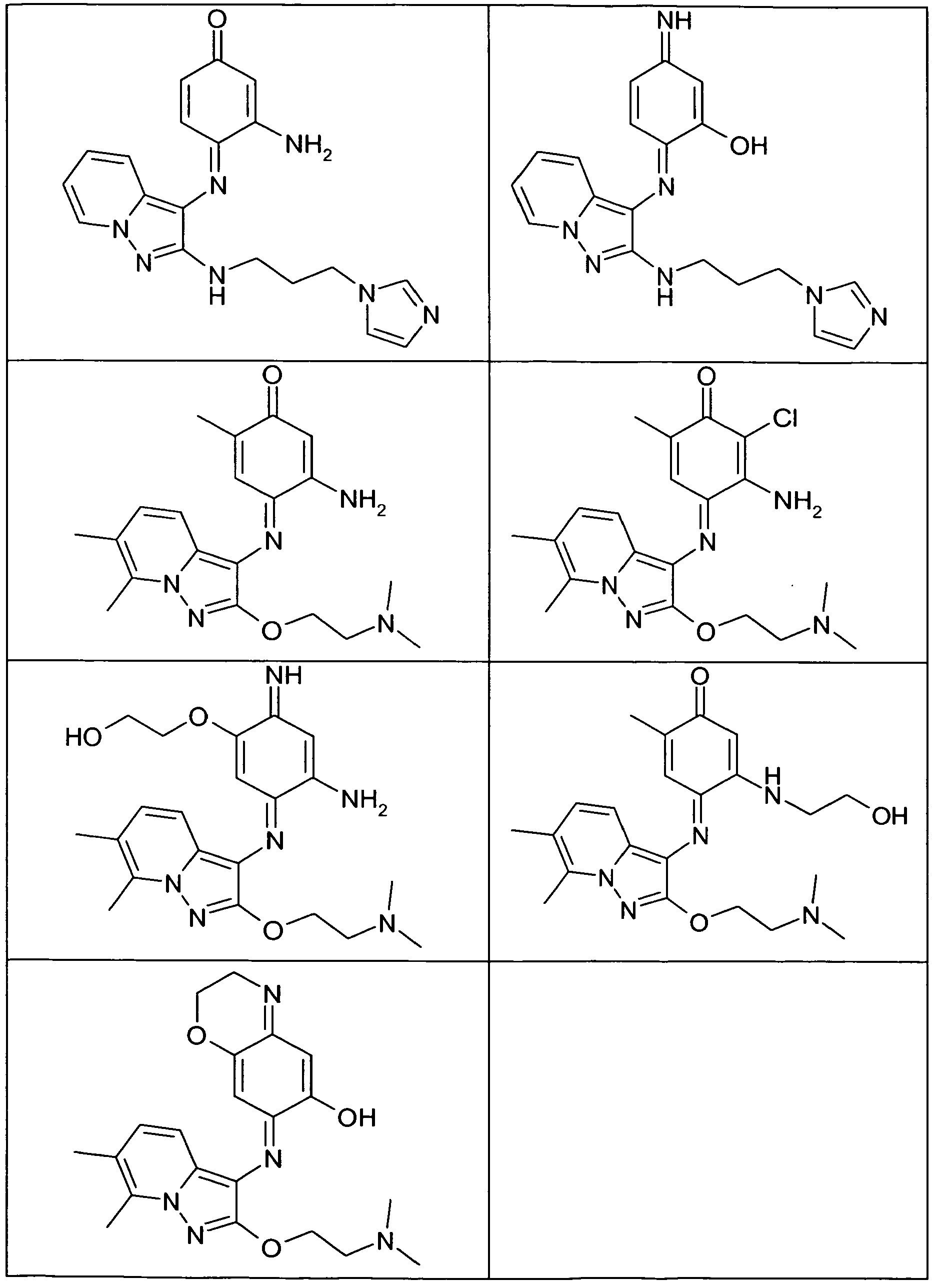

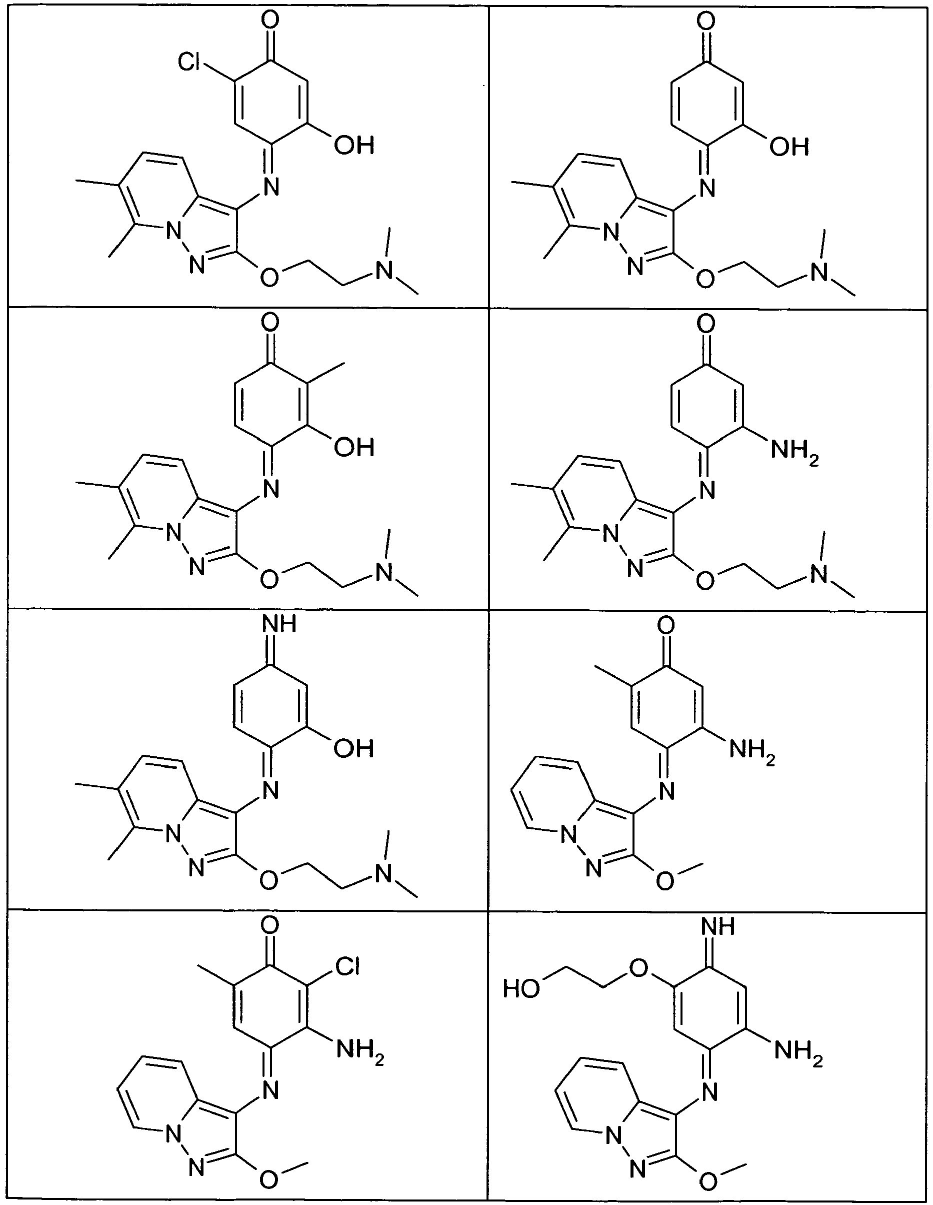

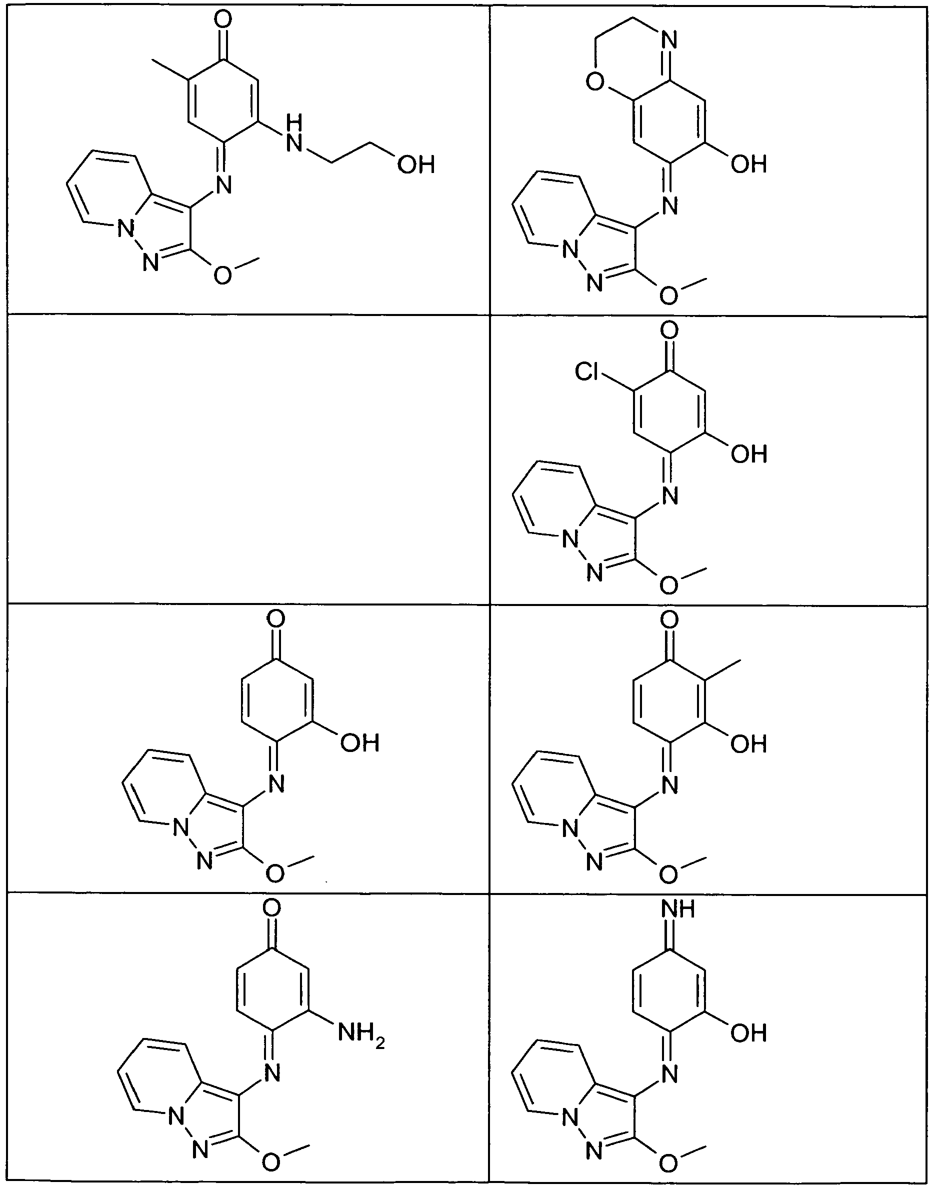

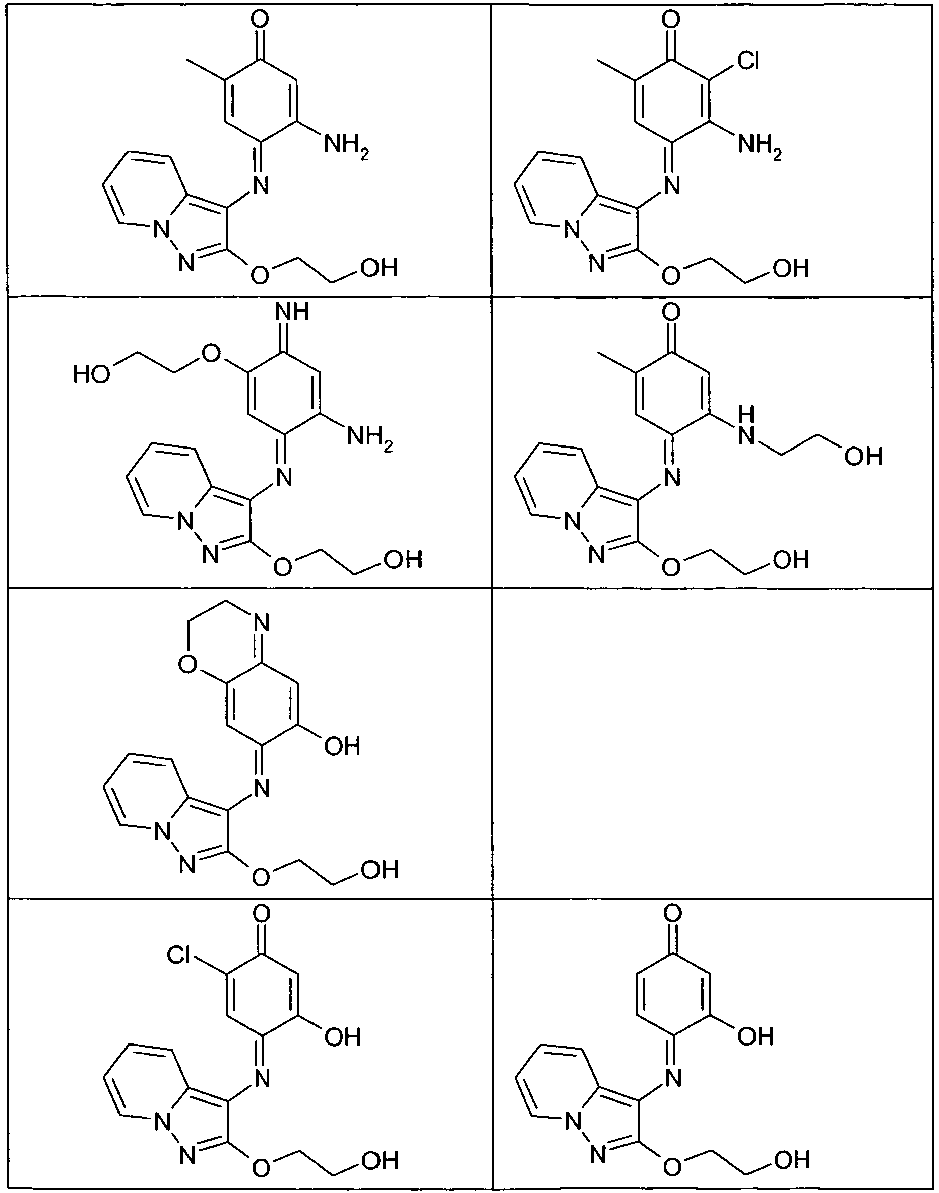

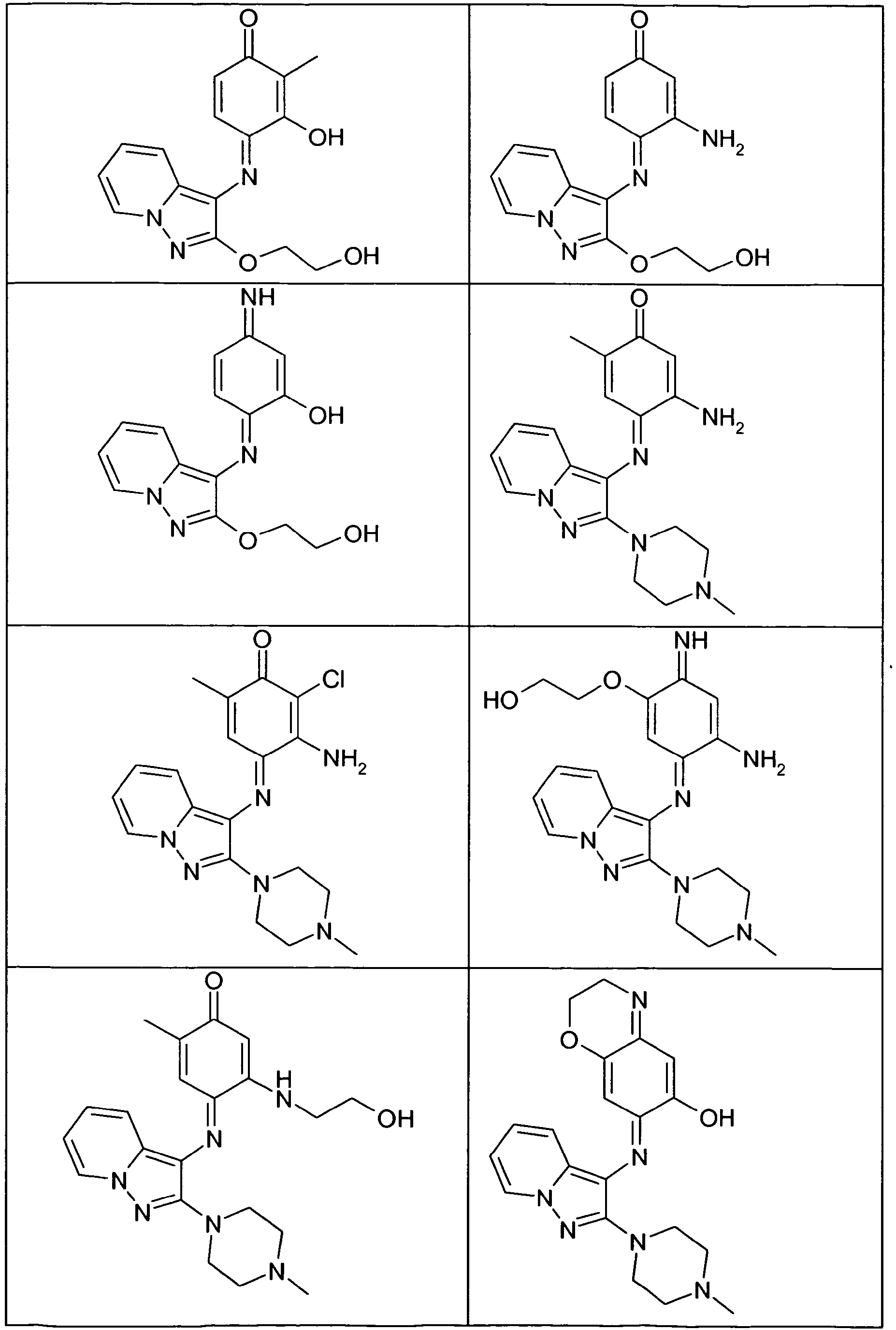

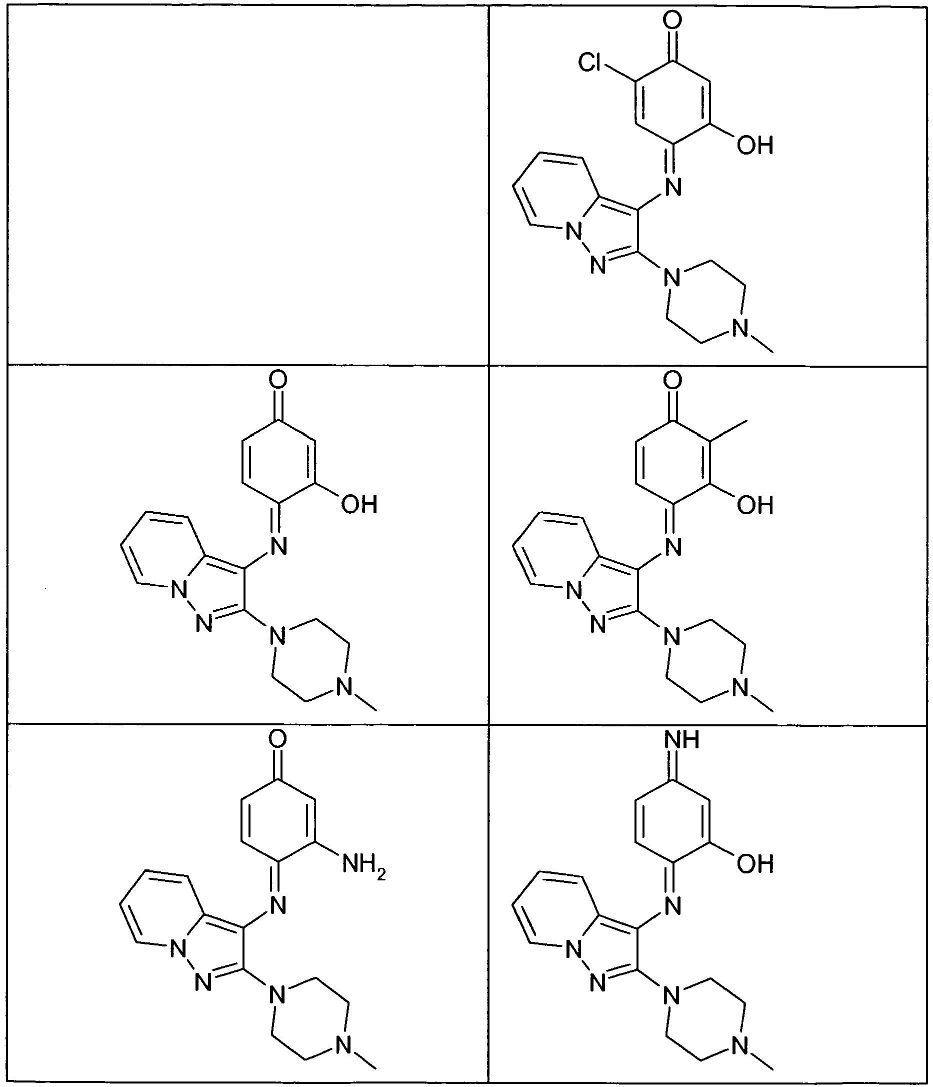

ainsi que leurs isomères, tautomères, mésomères, solvates et sels d'addition.

De préférence, les colorants de type azométhinique à motif pyrazolopyridine de formule (II) sont choisis parmi les composés suivants :

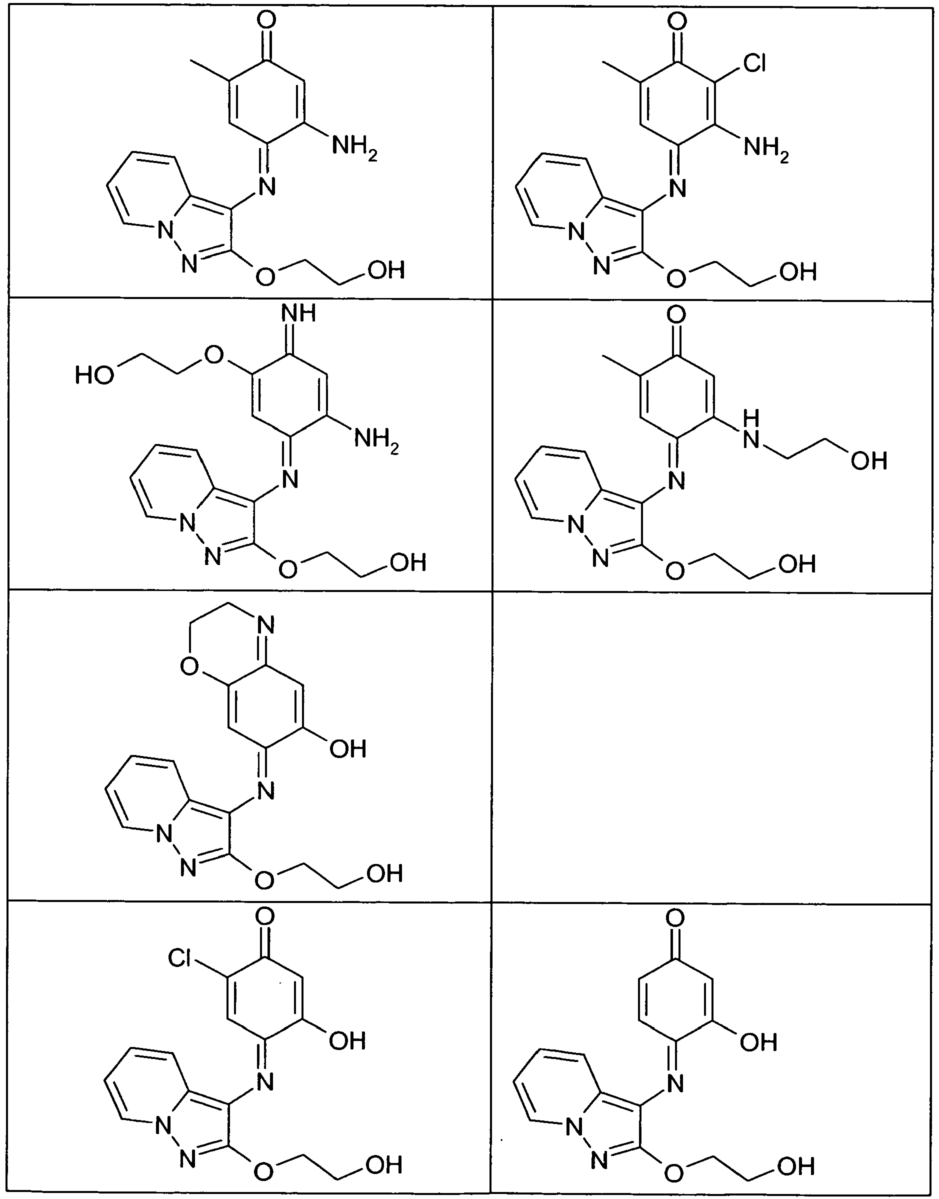

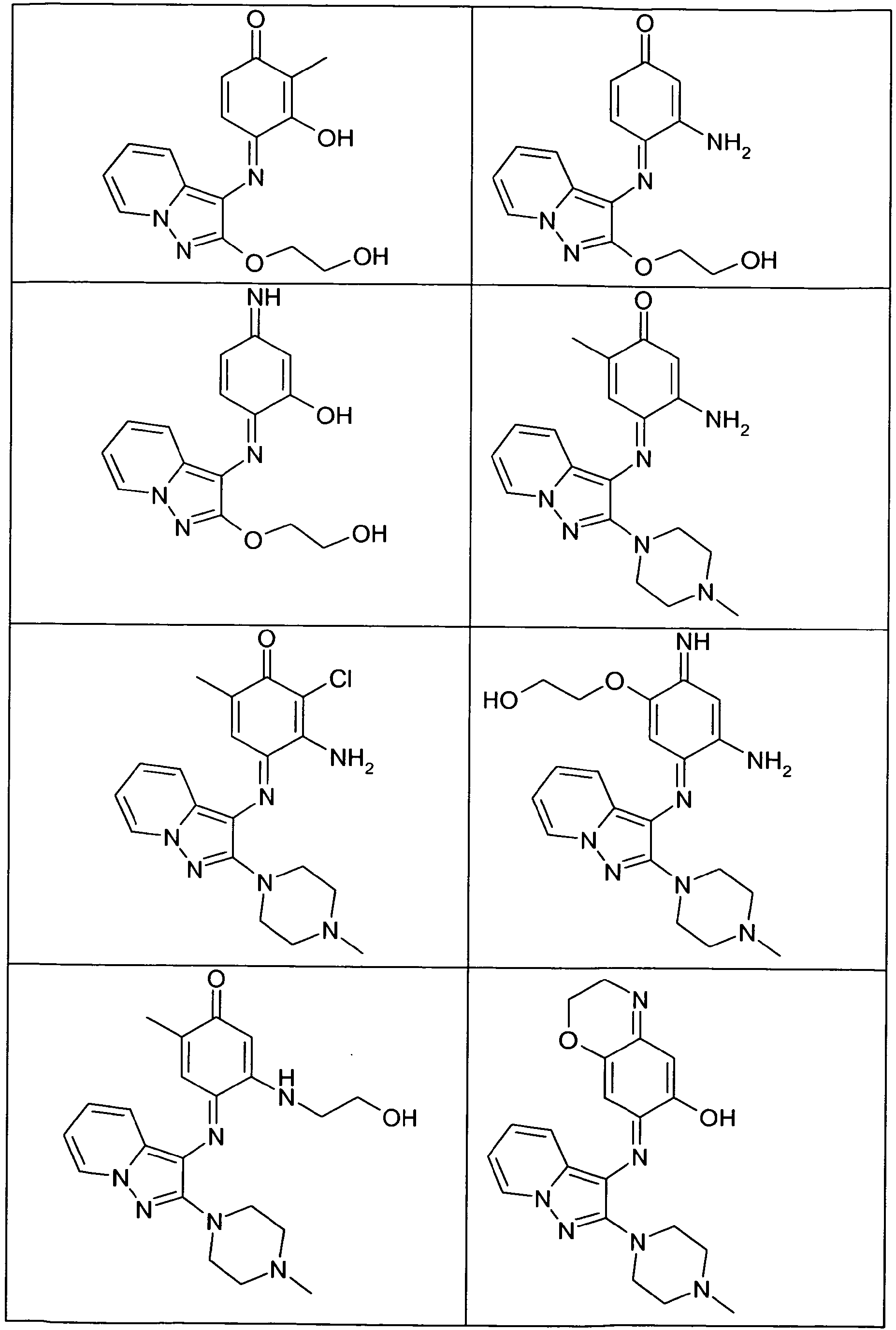

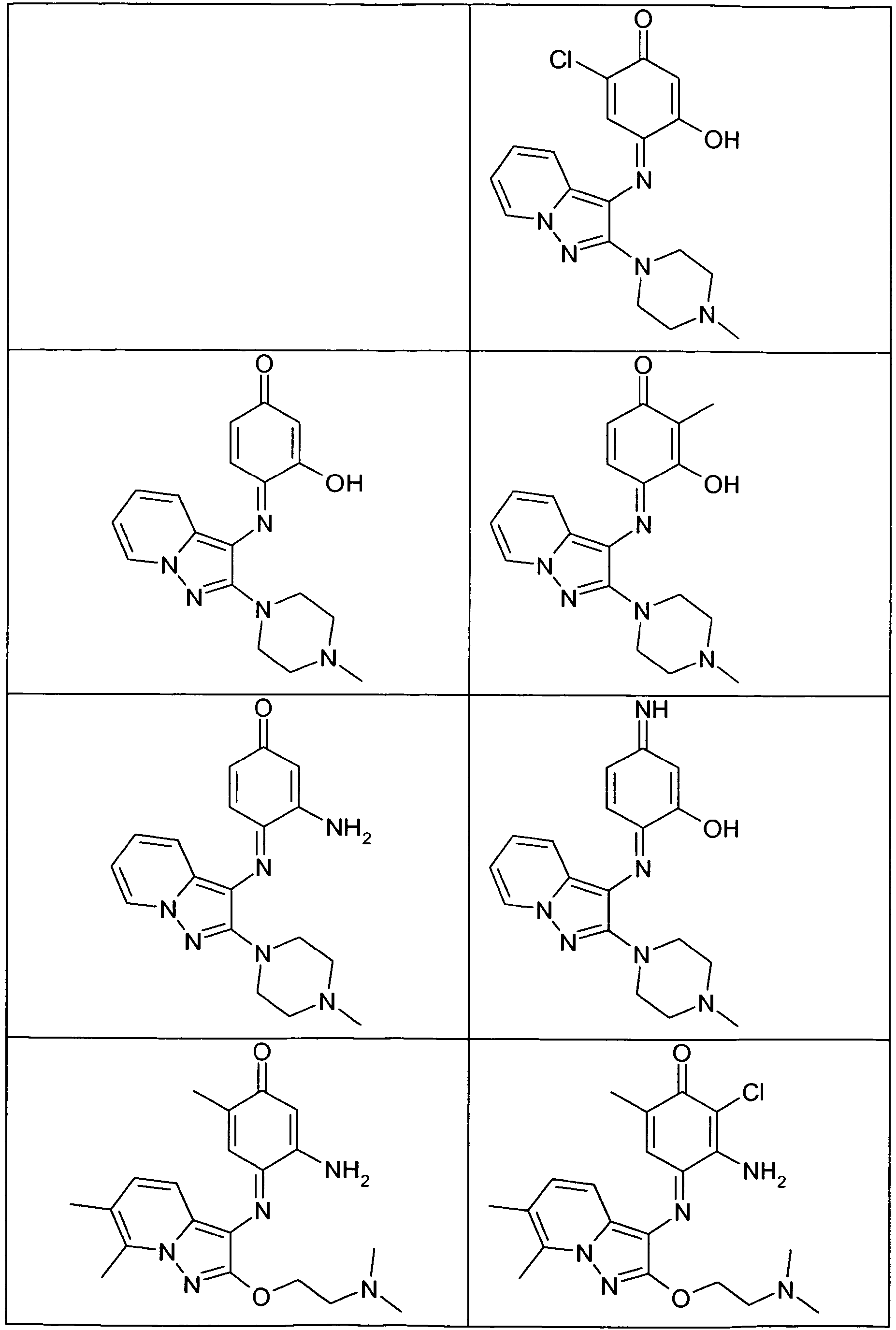

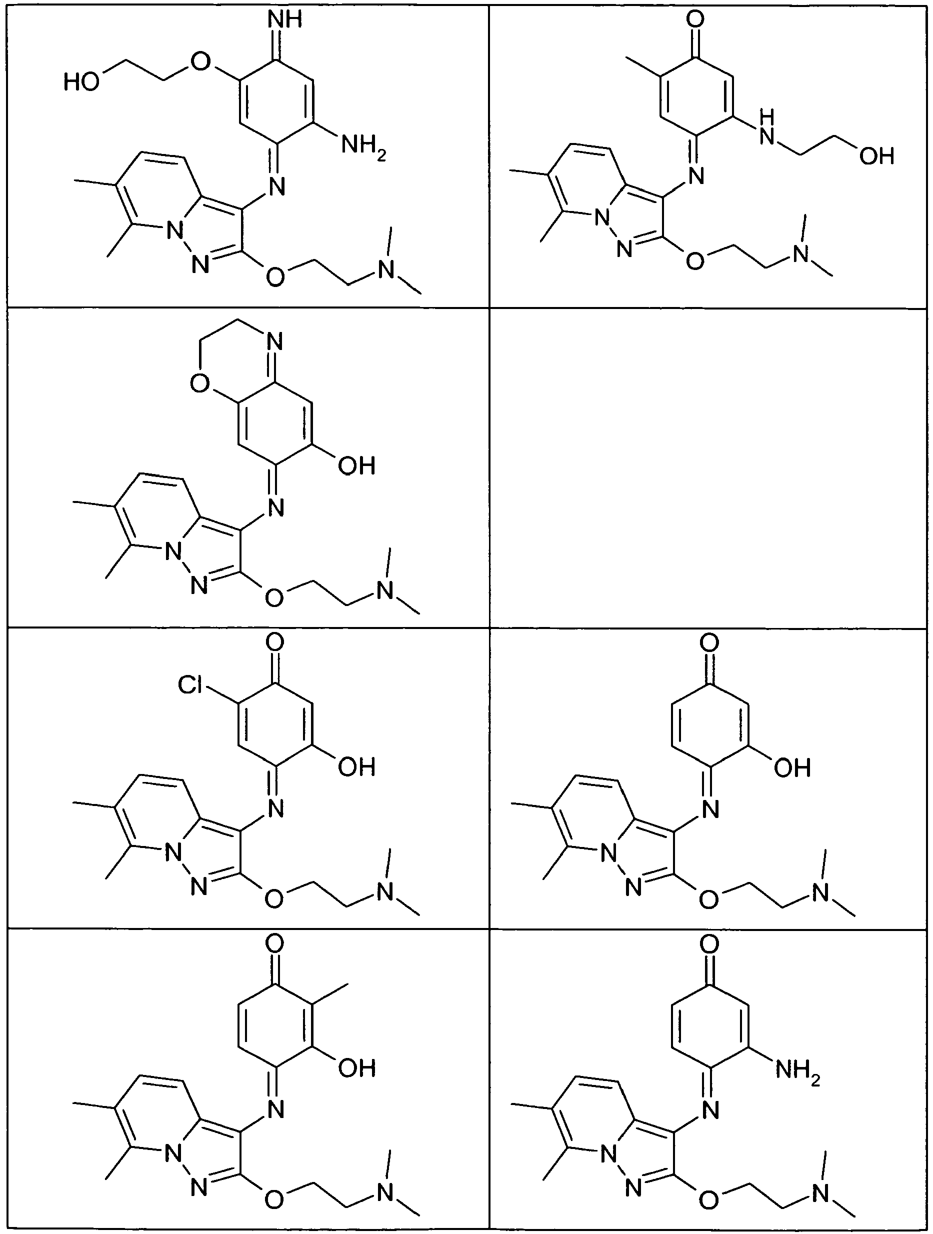

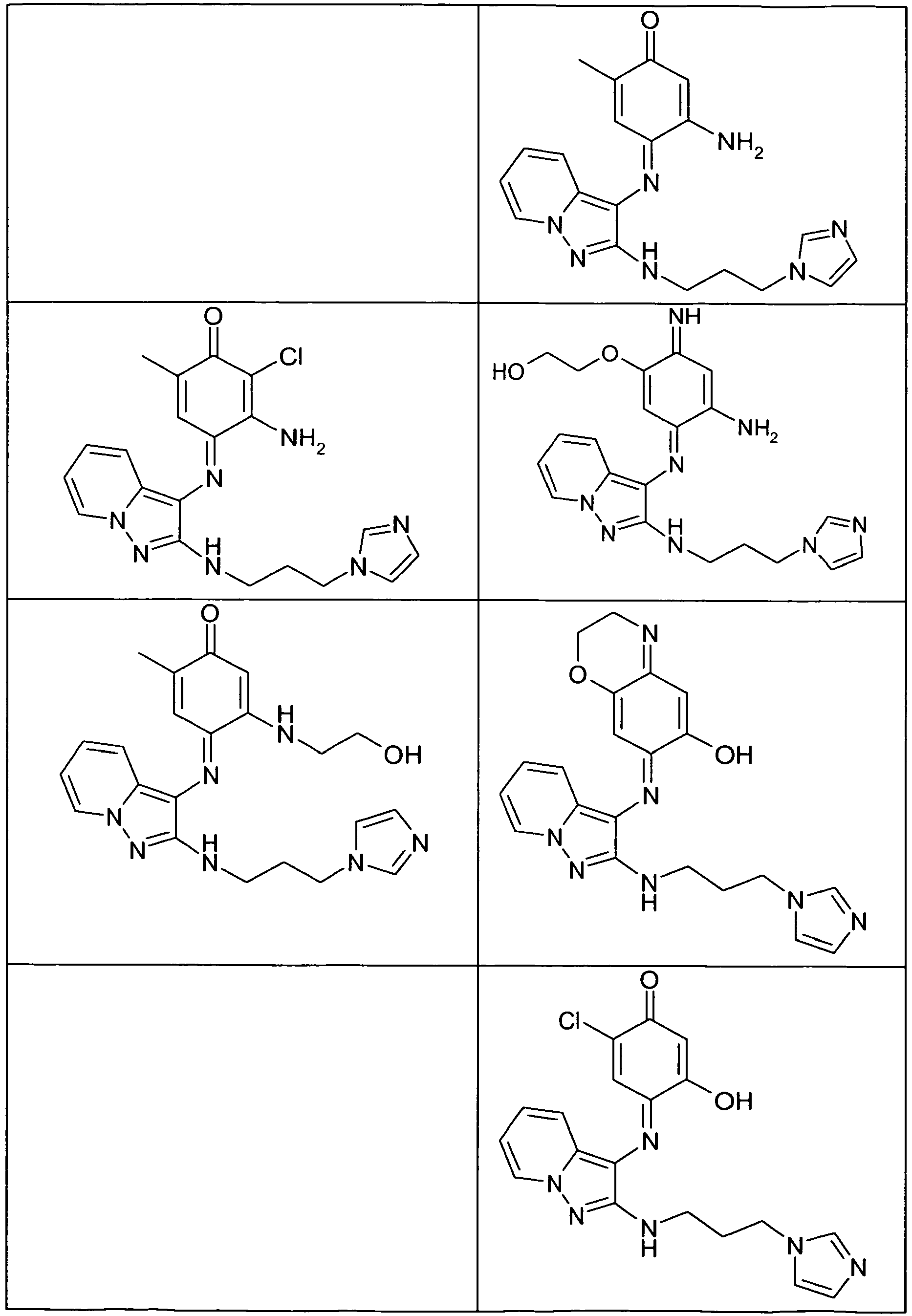

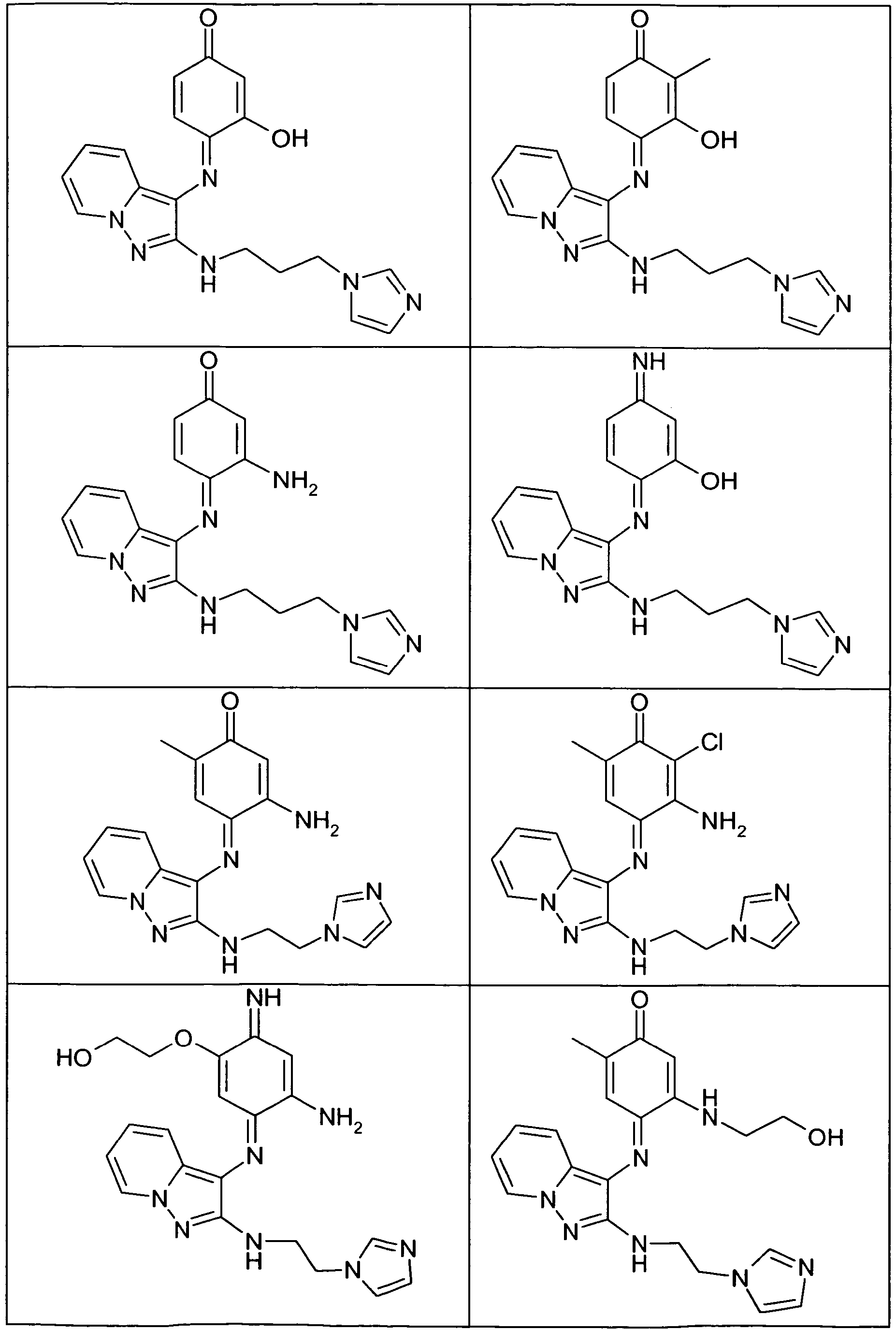

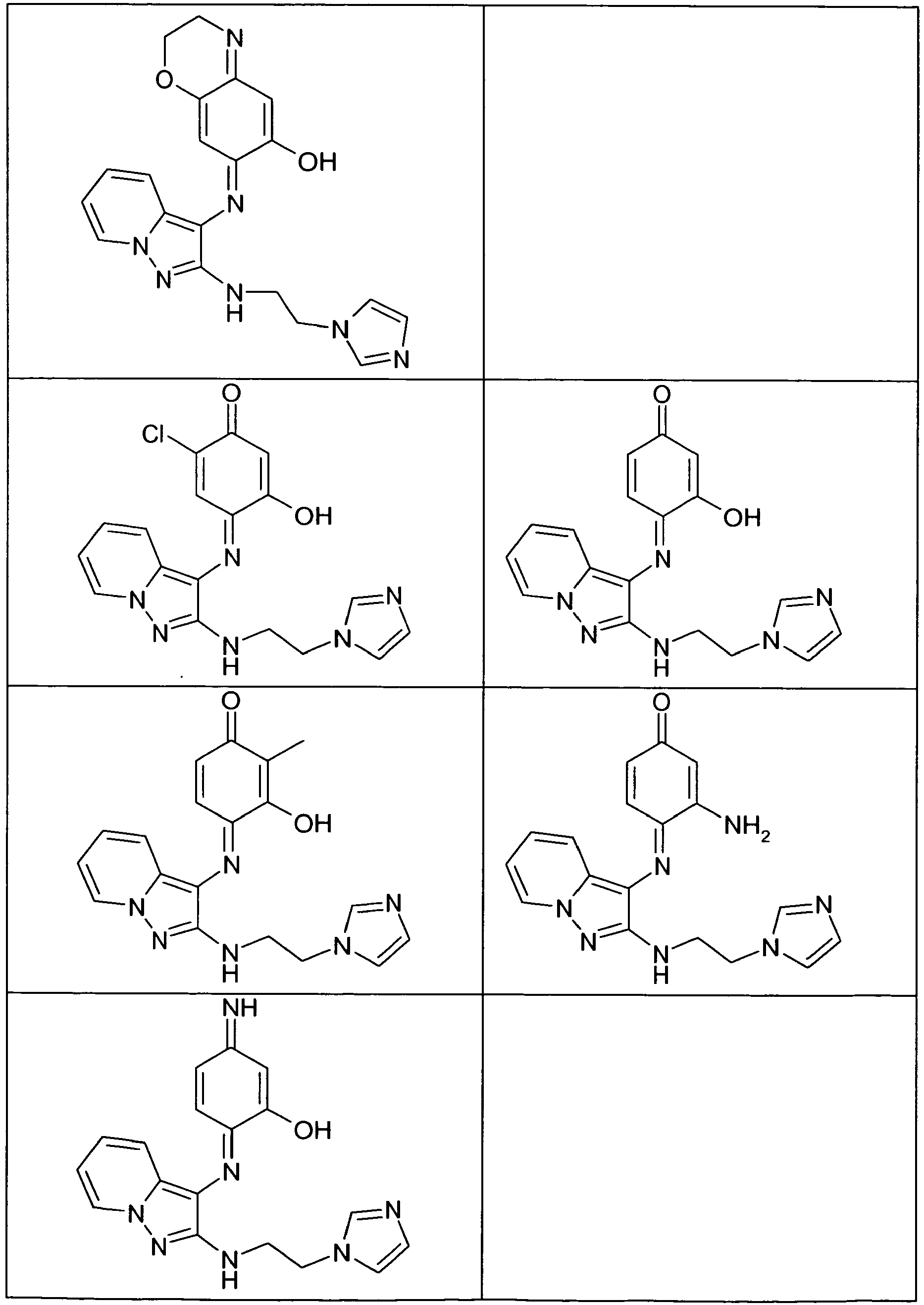

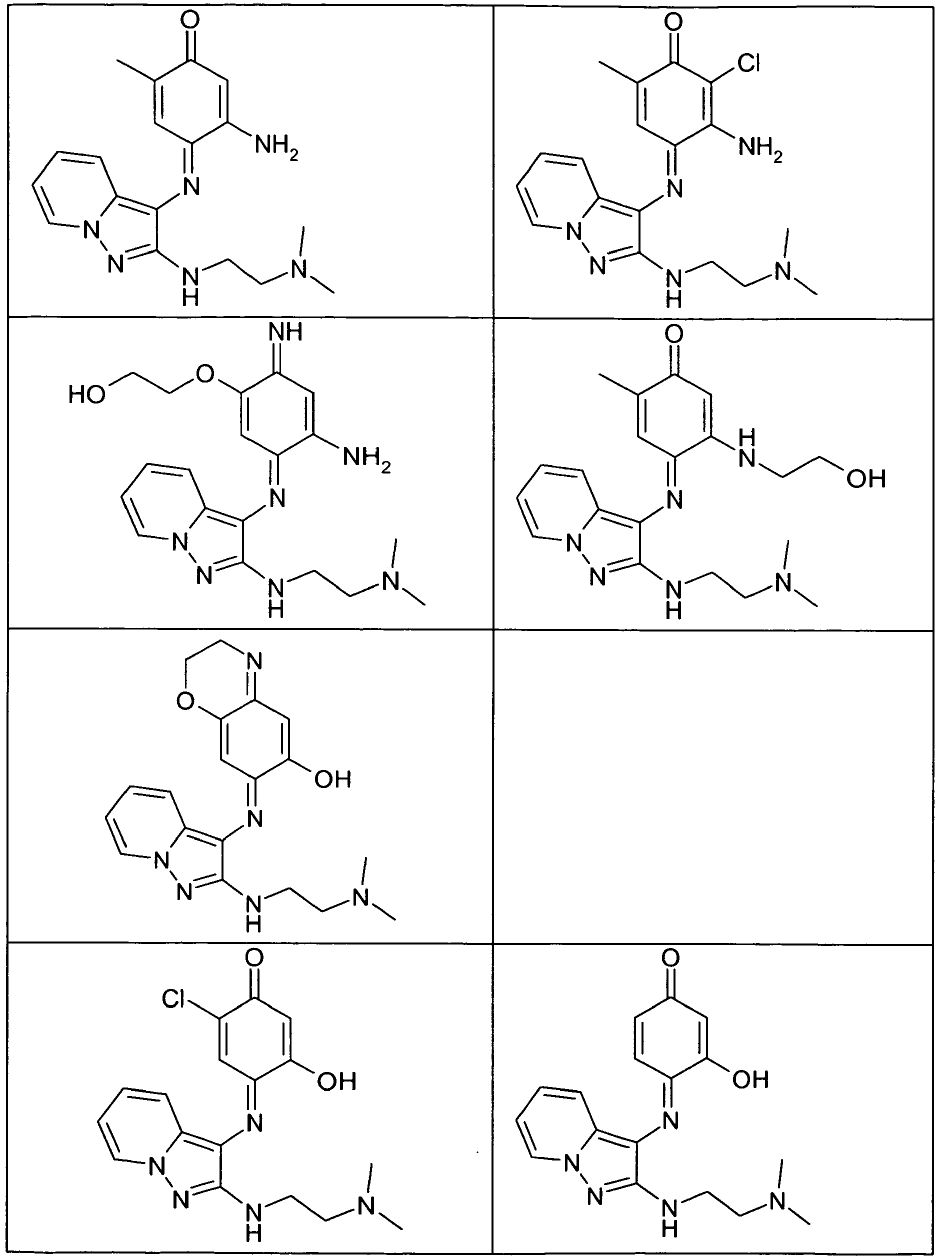

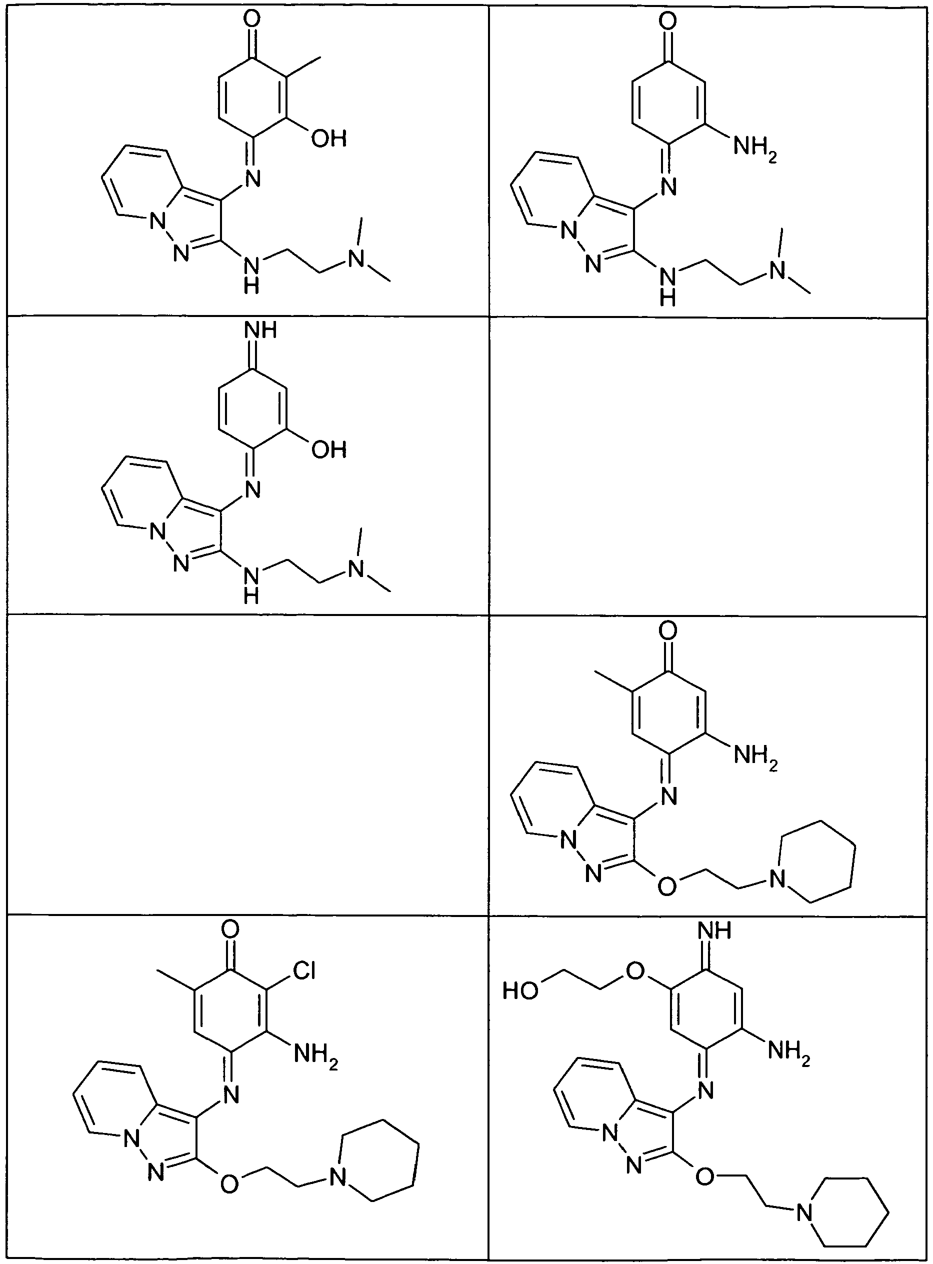

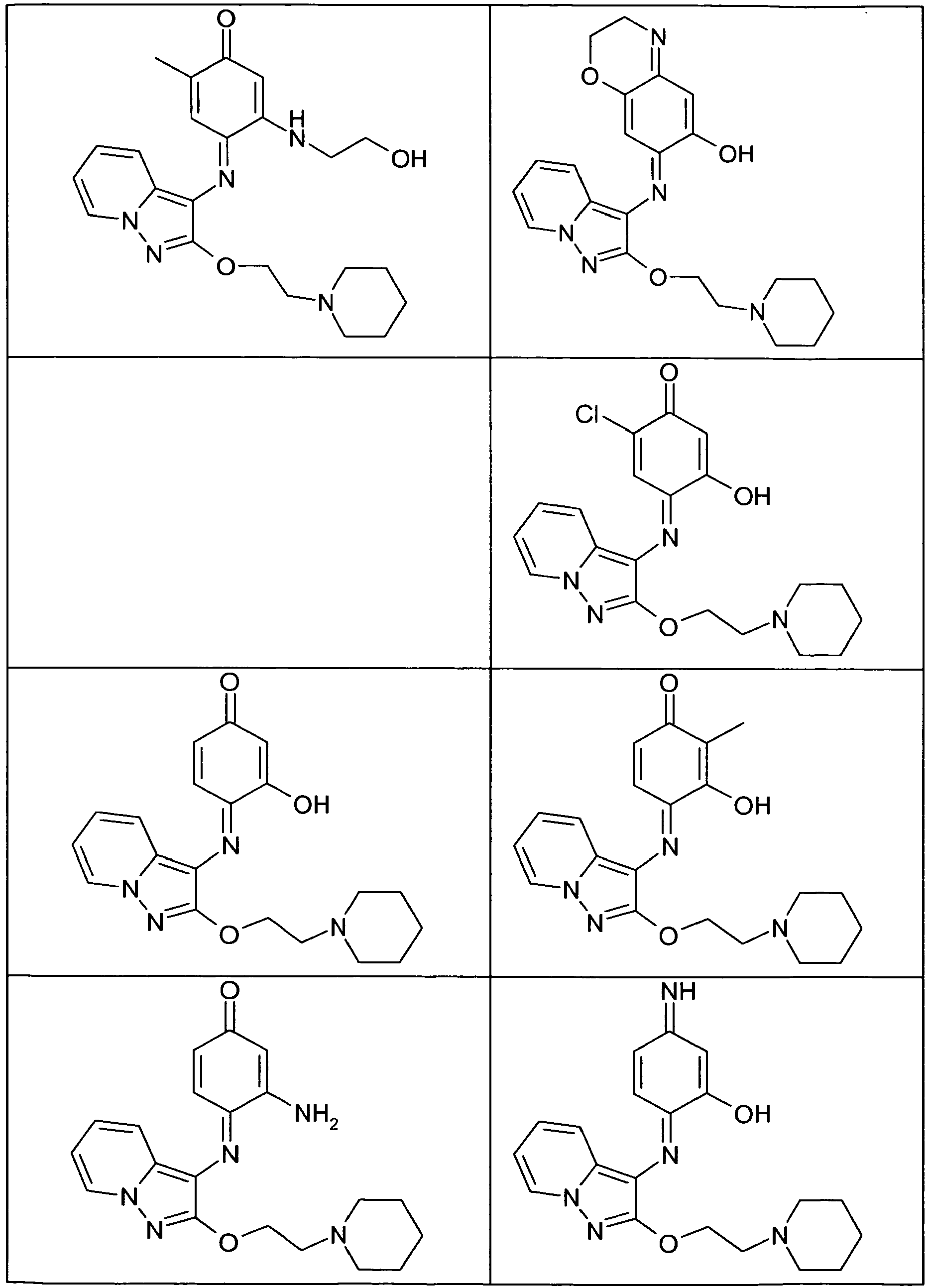

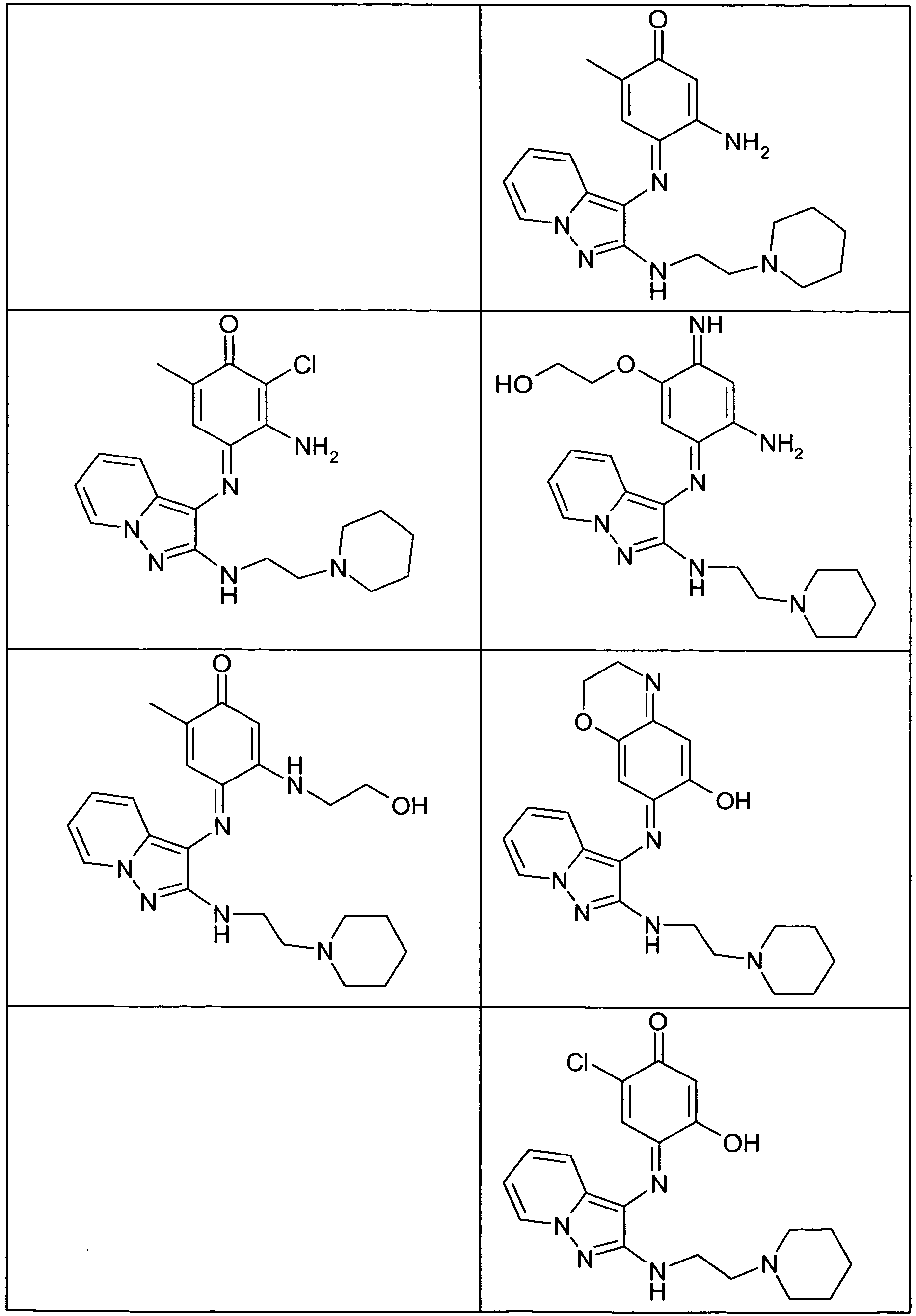

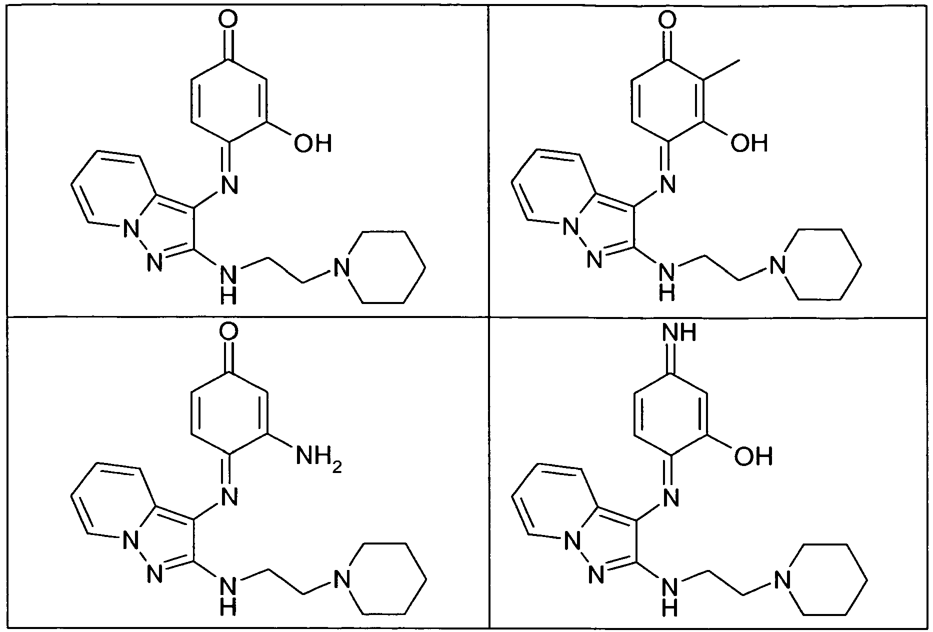

ainsi que leurs isomères, mésomères, tautomères, solvates et sels d'addition, et les composés leuco corresponants.

Selon un code de réalisation particulier, les colorants azométhiniques sont choisis parmi les composés de formule (IIa) ou (IIb) : 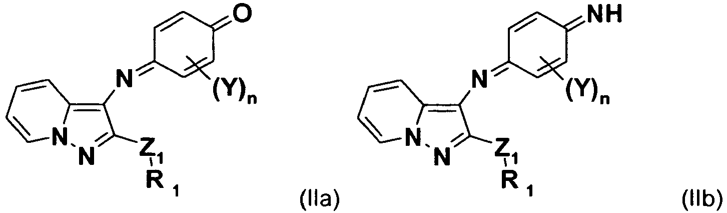 dans lesquelles R₁ et Z₁, Y et n sont tels que définis précédemment. Selon un mode de réalisation particulier, Z₁ est choisi par un atome oxygène ou un groupe NR₆. Lorsque Z₁ représente un atome d'oxygène, R₁ est de préférence un radical hydroxyalkyle ou un alkylamino subsitué ou non par un ou deux alkyle sur l'atome d'azote. Lorsque Z₁ est NR₆ alors R₁ peut former avec R₆ un cycle piperazinyle ou R₆ et l'hydrogène et R₁ est un radical alkyle subsitué par un radical imidazole.

Selon un autre mode de réalisation, n est 0, 1 ou 2, et Y est choisi parmi un radical hydroxy, alkyle, hydroxyalcoxy, ou halogène,

Les composés de formule (I) et/ou (II) peuvent être obtenus selon le mode opératoire ci-dessous :

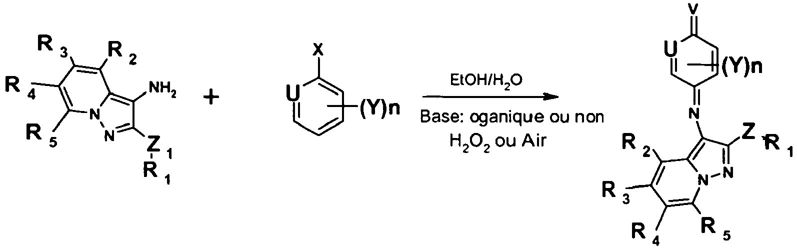

Dans un bécher, on pèse la base de type pyrazolopyridine que l'on dissout dans l'eau et/ou l'éthanol à température ambiante. On ajoute ensuite le coupleur, suivi d'une base telle que l'ammoniaque ou la soude, la potasse, un carbonate minéral tel que le carbonate de potassium, ou un acétate en présence d'oxydant. L'oxydant peut être de l'air, de l'eau oxygénée ou tout autre oxydant chimique. Le milieu réactionnel se colore dès l'ajout des deux derniers réactifs. Le milieu réactionnel ainsi obtenu est maintenu sous agitation pour une durée de 30 minutes à 24 heures. Le produit formé est filtré puis lavé à l'eau puis éventuellement à l'isopropyl l'éther. Le composé récupéré sous forme de poudre est séché à 20 °C sous vide jusqu'à poids constant. Dans le cas ou il n'y a pas de précipitation, le composé issu de cette réaction est récupéré par évaporation du solvant et éventuellement purifié sur colonne de silice. La caractérisation est réalisée par spectroscopie RMN et/ou spectrométrie de masse.

La présente invention a également pour objet une composition pour la coloration des fibres kératiniques comprenant, dans un milieu approprié pour la teinture, au moins un composé choisi parmi les composés de type leuco de formule (I), les colorants de type azométhinique à motif pyrazolopyridine de formule (II) correspondant aux composés de formule (I), leurs formes mésomères, isomères et,tautomères, ainsi que leurs sels d'addition avec un acide et leurs solvates, à l'exception du composé suivant : 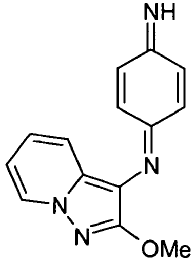 ainsi que ses formes mésomères isomères et tautomères, ses sels d'addition avec un acide et ses solvates.

Le ou les composés choisis parmi les composés de type leuco de formule (I), les colorants de type azométhinique à motif pyrazolopyridine de formule (II) correspondant aux composés de formule (I), leurs formes mésomères, isomères et tautomères ainsi que leurs sels d'addition avec un acide et leurs solvates représentent en général de 0,01 à 15%, plus particulièrement de 0,05 à 10% en poids par rapport au poids total de la composition.

La composition tinctoriale utile dans le cadre de l'invention peut de plus comprendre une base d'oxydation. Cette base d'oxydation peut être choisie parmi les bases d'oxydation classiquement utilisées en teinture d'oxydation, par exemple les para-phénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques et les dérivés de type pyrazolo[1,2a]pyrazol-1-one.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés de type pyrazolo[1,2a]pyrazol-1-one, on peut citer les composés comme le 2,3-diamino-6,7-dihydro,1H-5H-pyrazolo[1,2a]pyrazol-1-one.

La composition tinctoriale utile dans le cadre de l'invention peut contenir de plus un ou plusieurs coupleurs conventionnellement utilisés pour la teinture des fibres kératiniques. Parmi ces coupleurs, on peut notamment citer les méta-phénylènediamines, les méta-aminophénols, les méta-diphénols, les coupleurs naphtaléniques et les coupleurs hétérocycliques.

A titre d'exemple, on peut citer le 2-méthyl 5-aminophénol, le 5-N-(ß-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition tinctoriale utile dans le cadre de la présente invention, la ou les bases d'oxydation sont en général présentes en quantité comprise entre 0,001 à 10%, et plus préférentiellement de 0,005 à 6% en poids du poids total de la composition. Le ou les coupleurs sont en général présents en quantité comprise entre 0,001 et 10%, et plus préférentiellement de 0,005 à 6% en poids du poids total de la composition.

D'une manière générale, les sels d'addition avec un acide utilisables dans le cadre de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale utile dans le cadre de l'invention peut éventuellement comprendre au moins un colorant direct additionnel conventionnellement utilisé pour la teinture des fibres kératiniques. Celui-ci peut être choisi parmi les espèces cationiques ou non ioniques.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Il peut par exemple être choisi parmi les colorants benzéniques nitrés rouges ou orangés suivants : le 1-hydroxy-3-nitro-4-N-(γ-hydroxypropyl)amino benzène, le N-(β-hydroxyéthyl)amino-3-nitro-4-amino benzène, le 1-amino-3-méthyl-4-N-(β-hydroxyéthyl)amino-6-nitro benzène, le 1-hydroxy-3-nitro-4-N-(β-hydroxyéthyl)amino benzène, le 1,4-diamino-2-nitrobenzène, le 1-amino-2-nitro-4-méthylamino benzène, la N-(β-hydroxyéthyl)-2-nitro-paraphénylènediamine, le 1-amino-2-nitro-4-(β-hydroxyéthyl)amino-5-chloro benzène, la 2-nitro-4-amino-diphénylamine, le1-amino-3-nitro-6-hydroxybenzène, le 1-(β-aminoéthyl)amino-2-nitro-4-(β-hydroxyéthyloxy) benzène, le 1-(β,γ-dihydroxypropyl)oxy-3-nitro-4-(β-hydroxyéthyl)amino benzène, le 1-hydroxy-3-nitro-4-aminobenzène, le 1-hydroxy-2-amino-4,6-dinitrobenzène, le 1-méthoxy-3-nitro-4-(β-hydroxyéthyl)amino benzène, la 2-nitro-4'-hydroxydiphénylamine, le 1-amino-2-nitro-4-hydroxy-5-méthylbenzène.

Le colorant direct additionnel peut aussi être choisi parmi les colorants directs benzéniques nitrés jaunes et jaune-verts, on peut par exemple citer les composés choisis parmi : le 1-β-hydroxyéthyloxy-3-méthylamino-4-nitrobenzène, le 1-méthylamino-2-nitro-5-(β,γ-dihydroxypropyl)oxy benzène, le 1-(β-hydroxyéthyl)amino-2-méthoxy-4-nitrobenzène, le 1-(β-aminoéthyl)amino-2-nitro-5-méthoxy-benzène, le 1,3-di(β-hydroxyéthyl)amino-4-nitro-6-chlorobenzène, le 1-amino-2-nitro-6-méthyl-benzène, le 1-(β-hydroxyéthyl)amino-2-hydroxy-4-nitrobenzène, la N-(β-hydroxyéthyl)-2-nitro-4-trifluorométhylaniline, l'acide 4-(β-hydroxyéthyl)amino-3-nitro-benzènesulfonique, l'acide 4-éthylamino-3-nitro-benzoïque, le 4-(β-hydroxyéthyl)amino-3-nitro-chlorobenzène, le 4-(β-hydroxyéthyl)amino-3-nitro-méthylbenzène, le 4-(β,γ-dihydroxypropyl)amino-3-nitro-trifluorométhylbenzène, le 1-(β-uréidoéthyl)amino-4-nitrobenzène, le 1,3-diamino-4-nitrobenzène, le 1-hydroxy-2-amino-5-nitrobenzène, le 1-amino-2-[tris(hydroxymèthyl)méthyl]amino-5-nitrobenzène, le 1-(β-hydroxyéthyl)amino-2-nitrobenzène, le 4-(β-hydroxyéthyl)amino-3-nitrobenzamide.

On peut aussi mentionner les colorants directs benzéniques nitrés bleus ou violets, comme par exemple le 1-(β-hydroxyéthyl)amino-4-N,N-bis-(β-hydroxyéthyl)amino 2-nitrobenzène, le 1-(γ-hydroxypropyl)amino 4-N,N-bis-(β-hydroxyéthyl)amino 2-nitrobenzène, le 1-(β-hydroxyéthyl)amino 4-(N-méthyl, N-β-hydroxyéthyl)amino 2-nitrobenzène, le 1-(β-hydroxyéthyl)amino 4-(N-éthyl, N-β-hydroxyéthyl)amino 2-nitrobenzène, le 1-(β,γ-dihydroxypropyl)amino 4-(N-éthyl, N-β-hydroxyéthyl)amino 2-nitrobenzène, les 2-nitroparaphénylènediamines de formule (III) suivante : 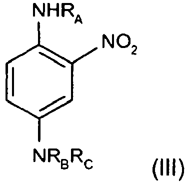 dans laquelle :
- R_{B} représente un radical alkyle en C₁-C₄, un radical β-hydroxyéthyle ou β-hydroxypropyle ou β-hydroxypropyle ;
- R_{A} et R_{C}, identiques ou différents, représentent un radical β-hydroxyéthyle, β-hydroxypropyle, γ-hydroxypropyle, ou β,γ-dihydroxypropyle, l'un au moins des radicaux R_{B}, R_{C} ou R_{A} représentant un radical γ-hydroxypropyle et R_{B} et R_{C} ne pouvant désigner simultanément un radical β-hydroxyéthyle lorsque R_{B} est un radical γ-hydroxypropyle, telles que celles décrits dans le brevet français FR 2 692 572.

Parmi les colorants directs azoïques utilisables selon l'invention, on peut citer les colorants azoïques cationiques décrits dans les demandes de brevets WO 95/15144, WO 95/01772, EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi ces composés on peut tout particulièrement citer les colorants suivants : le chlorure de 1,3-diméthyl-2-[[4-(diméthylamino)phényl]azo]-1H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{ème} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ,ainsi que les composés suivants : la 1-N-méthylmorpholiniumpropylamino-4-hydroxyanthraquinone, la 1-aminopropylamino-4-méthylaminoanthraquinone, la 1-aminopropylamino-anthraquinone, la 5-β-hydroxyéthyl-1,4-diaminoanthraquinone, la 2-aminoéthylamino-anthraquinone, la 1,4-bis-(β,γ-dihydroxypropylamino)anthraquinone.

Parmi les colorants aziniques, on peut citer les composés suivants : Basic Blue 17, Basic Red 2.

Parmi les colorants triarylméthaniques utilisables selon l'invention, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques utilisables selon l'invention, on peut citer les composés suivants : la 2-β-hydroxyéthlyamino-5-[bis-(β-4'-hydroxyéthyl)amino]anilino-1,4-benzoquinone, la 2-β-hydroxyéthylamino-5-(2'-méthoxy-4'-amino)anilino-1,4-benzoquinone, la 3-N(2'-chloro-4'-hydroxy)phényl-acétylamino-6-méthoxy-1,4-benzoquinone imine, la 3-N(3'-chloro-4'-méthylamino)phényl-uréido-6-méthyl-1,4-benzoquinone imine, la 3-[4'-N-(éthyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants : le chlorure de 2-((E)-{(E)-[(1,3-diméthyl-1,3-dihydro-2H-imidazol-2-ylidène)hydrazono]méthyl}diazényl)-1,3-diméthyl-1H-imidazol-3-ium ; le chlorure de 2-{(E)-[(1Z)-N-(1,3-diméthyl-1,3-dihydro-2H-imidazol-2-ylidène)éthanehydrazonoyl]diazényl}-1,3-diméthyl-1H-imidazol-3-ium; le chlorure de 4-méthoxy-2-((E)-{(1E)-1-[(2E)-(4-méthoxy-1-méthylpyridin-2(1H)-ylidène)hydrazono] éthyl}diazényl)-1-méthylpyridinium; le chlorure de 1-méthyl-2-((E)-{(1E)-1-[(2E)-(1-méthylpyridin-2(1H)-ylidène)hydrazono]éthyl}diazényl)pyridinium ; le chlorure de 1-(2-hydroxyéthyl)-2-[(E)-((1E)-1-{(2E)-[1-(2-hydroxyéthyl)pyridin-2(1H)-ylidène]hydrazono}éthyl)diazényl]pyridinium ; le chlorure de 1-méthyl-2-((E)-{(E)-[(2Z)-(1-méthylpyridin-2(1H)-ylidène)hydrazono]méthyl}diazényl)pyridinium ; l'acétate de 1-(2-hydroxyéthyl)-2-[(E)-((E)-{(2E)-[1-(2-hydroxyéthyl)pyridin-2(1H)-ylidène]hydrazono}méthyl)diazényl]pyridinium.

Parmi les colorants directs additionnels naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

S'ils sont présents, la teneur en colorants directs additionnels dans la composition varie en général de 0,001 à 20%, et de préférence de 0,01 à 10% en poids par rapport au poids de la composition.

Le milieu approprié pour la teinture, appelé aussi support de teinture, comprend généralement de l'eau ou un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant de 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants usuels décrits ci-dessus, s'ils sont présents, représentent habituellement de 1 à 40% en poids, plus préférentiellement de 5 à 30% en poids, par rapport au poids total de la composition.

La composition tinctoriale utile dans le cadre de l'invention peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci-dessus sont en général présents en quantité comprise pour chacun d'eux entre 0,01 et 20% en poids par rapport au poids de la composition.

Bien entendu, l'homme de l'art veillera à choisir ce ou ces éventuels composés complémentaires de manière telle que les propriétés avantageuses attachées intrinsèquement à la composition tinctoriale utile dans le cadre de l'invention ne soient pas, ou substantiellement pas, altérées par la ou les adjonctions envisagées.

Le pH de la composition tinctoriale utile dans le cadre de l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques. La modification du pH dans ces intervalles favorisera la formation des composés (I) ou (II).

Parmi les agents acidifiants, on peut citer, à titre d'exemple, les acides minéraux comme par exemple l'acide chlorhydrique, l'acide nitrique, l'acide sulfurique ou les acides organiques comme par exemple les composés comprenant au moins une fonction acide carboxylique comme l'acide acétique, l'acide tartrique, l'acide citrique, l'acide lactique, une fonction acide sulfonique, une fonction acide phosphonique ou une fonction acide phosphorique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 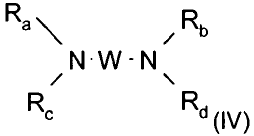 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

Les composés de formule (II) peuvent être obtenus à partir des composés de formule (I) par réaction avec l'oxygène de l'air ou par action d'un agent oxydant pouvant être n'importe quel agent oxydant utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydoréductases à 2 électrons telles que les uricases et les oxygénases à 4 électrons comme les laccases.

L'agent oxydant sera aussi nécessaire pour l'obtention d'un éclaicissement simultané des fibres kératiniques (coloration éclaircissante) et/ou lorsque la composition contient des bases d'oxydation ou des coupleurs.

La composition tinctoriale utile dans le cadre de l'invention peut se présenter sous des formes diverses, telles que sous forme de liquides, de crèmes, de gels, ou sous toute autre forme appropriée pour réaliser une teinture des fibres kératiniques, et notamment des cheveux humains.

La coloration obtenue dépend des composés qui sont appliqués sur les fibres kératiniques. La coloration est plus intense lorsque la totalité de ces composés sont sous la forme de colorants de type azométhinique à motif pyrazolopyridine, c'est-à-dire qu'ils sont de formule (II). En favorisant la formation des composés de formule (I) à partir des composés de formule (II), on peut réduire l'intensité de la coloration jusqu'à la faire disparaître.

La présente invention a également pour objet les composés choisis parmi les composés de type leuco de formule (I), les colorants de type azométhinique à motif pyrazolopyridine de formule (II) tels que définis précédemment, ainsi que leurs formes mésomères isomères et tautomères, leurs sels d'addition avec un acide et leurs solvates ; à l'exception du composé suivant : 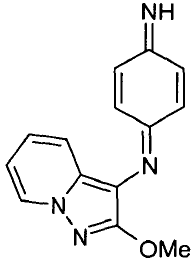 ainsi que ses formes mésomères, ses sels d'addition avec un acide et ses solvates.

Le procédé de l'invention comprend l'application sur les fibres kératiniques d'au moins une composition comprenant au moins un composé choisi parmi les composés de type leuco de formule (I), les colorants de type azométhinique à motif pyrazolopyridine de formule (II) correspondant aux composés de formule (I) tels que définis précédemment, ainsi que leurs formes mésomères isomères et tautomères, leurs sels d'addition avec un acide et leurs solvates.

Selon un mode de réalisation préféré, la composition appliquée comprend des composés de formule (II), (IIa) et (IIb).

Lorsque l'agent oxydant est mis en oeuvre, il peut être présent dans la composition de l'invention. Il peut aussi être appliqué séparément, en pré ou post traitement.

L'application de la composition de l'invention peut être ou non suivie d'un rinçage.

Le temps de pose de la composition tinctoriale est généralement compris entre 3 et 60 minutes, de préférence entre 5 et 40 minutes, encore plus préférentiellement entre 10 et 30 minutes.

La température d'application est généralement mise en oeuvre à la température ambiante, de préférence entre 25 et 55°C.

La présente invention a également pour objet un dispositif à plusieurs compartiments ou kit permettant de mettre en oeuvre le procédé pour la coloration des fibres kératiniques décrit ci-dessus.

Le dispositif à plusieurs compartiments de l'invention contient dans un premier compartiment une composition comprenant au moins un composé de formule (l), (ll), (IIa) et (IIb). et un deuxième compartiment qui comprend un agent oxydant, et éventuellement un composé de formule (II), (IIa) et (IIb)., un agent alcalin.

Ce dispositif peut être équipé d'un moyen permettant de délivrer sur les cheveux le mélange souhaité, tel que les dispositifs décrits dans le brevet FR-2586913.

Les exemples qui suivent servent à illustrer l'invention sans toutefois présenter un caractère limitatif.

### EXEMPLES

### Exemples de synthèse

### Exemple 1

### Synthèse du 2-({(3E)-4-amino-6-imino-3-[(2-méthoxypyrazolo[1,5-a]pyridin-3-yl)imino]cyclohexa-1,4-dièn-1-yl}oxy)éthanol

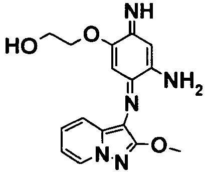

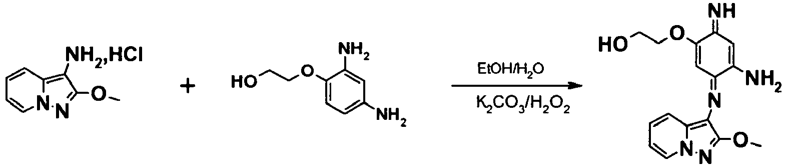

Dans un ballon monocol de 100 ml, on introduit 5 mmoles de dichlorhydrate de 2-méthoxypyrazolo[1,5-a]pyridin-3-amine que l'on dissout dans 20 ml d'éthanol.

A cette solution, on ajoute 5 mmoles de coupleur 2-(2,4-diaminophénoxy)éthanol et une solution de 20 mmoles de carbonate de potassium dans 3 ml d'eau.

On ajoute ensuite 100 microlitres d'eau oxygénée 9 volumes puis l'agitation est maintenue pendant 24 heures à température ambiante.

Après dilution avec 100 ml d'eau le composé attendu est isolé par filtration, lavé à l'eau puis séché sous vide à 30 °C en présence de desséchant.

337 mg de produit attendu sont ainsi isolés sous forme de poudre marron foncé.

### Analyse par spectrométrie de masse

L'ion quasi moléculaire [M+H]+ de la molécule attendue C₁₆H₁₇N₅O₃ est principalement détecté.

### Exemple 2

### Synthèse du 2-[(3-{[(1E)-2-amino-5-(2-hydroxyethoxy)-4-iminocyclohexa-2,5-dien-1-ylidene]amino}pyrazolo[1,5-a]pyridin-2-yl)oxy]ethanol

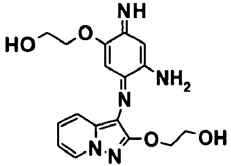

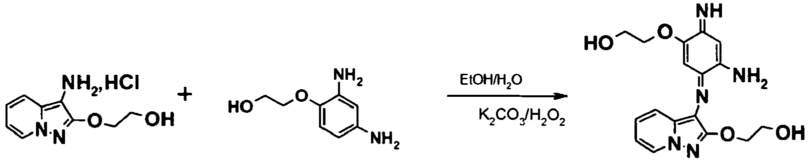

Dans un ballon monocol de 100 ml, on introduit 4,35 mmoles de chlorhydrate de 2-[(3-aminopyrazolo[1 ,5-a]pyridin-2-yl)oxy]éthanol que l'on dissout dans 25 ml d'éthanol.

A cette solution, on ajoute 4,35 mmoles de coupleur 2-(2,4-diaminophénoxy)éthanol et une solution de 17,41 mmoles de carbonate de potassium et 3 ml d'eau.

On ajoute ensuite 100 microlitres d'eau oxygénée à 20 volumes puis l'agitation est maintenue pendant 24 heures à température ambiante.

Après dilution avec 100 ml d'eau le composé attendu est isolé par filtration, lavé à l'eau puis séché sous vide à 30 °C en présence de desséchant.

Le produit isolé marron foncé (392,6 mg) correspond au produit attendu.

### Analyse par spectrométrie de masse

L'ion quasi moléculaire [M+H]+ de la molécule attendue C₁₇H₁₉N₅O₄ est principalement détecté.

### Exemple 3

### Synthèse du 2-({(3E)-4-amino-3-[(2-{[2-(diméthylamino)éthyl]amino}pyrazolo[1,5-a]pyridin-3-yl)imino]-6-iminocyclohexa-1,4-dièn-1-yl}oxy)éthanol

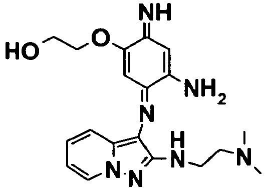

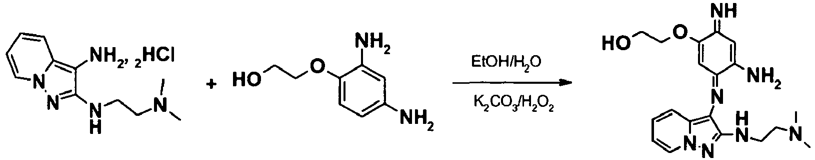

Dans un ballon monocol de 100 ml, on introduit 3,04 mmoles de dichlrohydrate de N2-[2-(diméthylamino)éthyl]pyrazolo[1,5-a]pyridine-2,3-diamine que l'on dissout dans 25 ml d'éthanol.

A cette solution, on ajoute 3,04 mmoles de coupleur 2-(2,4-diaminophénoxy)éthanol et une solution de 18,24 mmoles de carbonate de potassium et 3 ml d'eau.

On ajoute ensuite 100 microlitres d'eau oxygénée à 20 volumes puis l'agitation est maintenue pendant 24 heures à température ambiante.

Après dilution avec 100 ml d'eau le composé attendu est isolé par filtration, lavé à l'eau puis séché sous vide à 40 °C en présence de desséchant.

On isole ainsi le produit attendu sous forme de solide marron foncé (116,4 mg).

### Analyse par spectrométrie de masse

La molécule attendue C₁₉H₂₅N₇O₂ est principalement détectée à m/z, ESP+=384 et 192.

### Exemple 4

### Synthèse du (4E)-4-{[2-(2-hydroxyéthoxy)pyrazolo[1,5-a]pyridin-3-yl]imino}-5-[(2-hydroxyéthyl)amino]-2-méthylcyclohexa-2,5-dièn-1-one

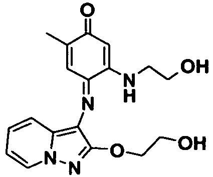

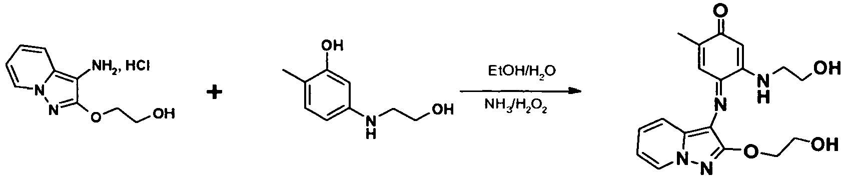

Dans un ballon monocol de 100 ml on introduit 5 mmoles de chlorhydrate de 2-[(3-aminopyrazolo[1 ,5-a]pyridin-2-yl)oxy]éthanol que l'on dissout dans 2 ml d'éthanol et 8 ml d'eau.

A cette solution, on ajoute 5 mmoles de coupleur 5-[(2-hydroxyéthyl)amino]-2-méthylphénol, 1 ml d'ammoniaque à 20 % de telle manière à obtenir un pH = 9,5 et 1 ml d'eau oxygénée comme oxydant.

Le milieu réactionnel (solution violette) est agité 30 minutes à température ambiante et le composé attendu est contrôlé par spectrométrie IC/MS.

### Analyse par spectrométrie de masse

La molécule attendue C₁₈H₂₀N₄O₄ est principalement détectée.

### Exemples de teinture

### Exemples 1 et 2 de teinture en milieu acide

Les compositions tinctoriales suivantes sont préparées :

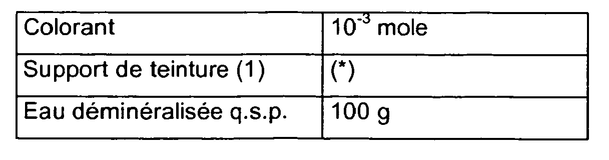

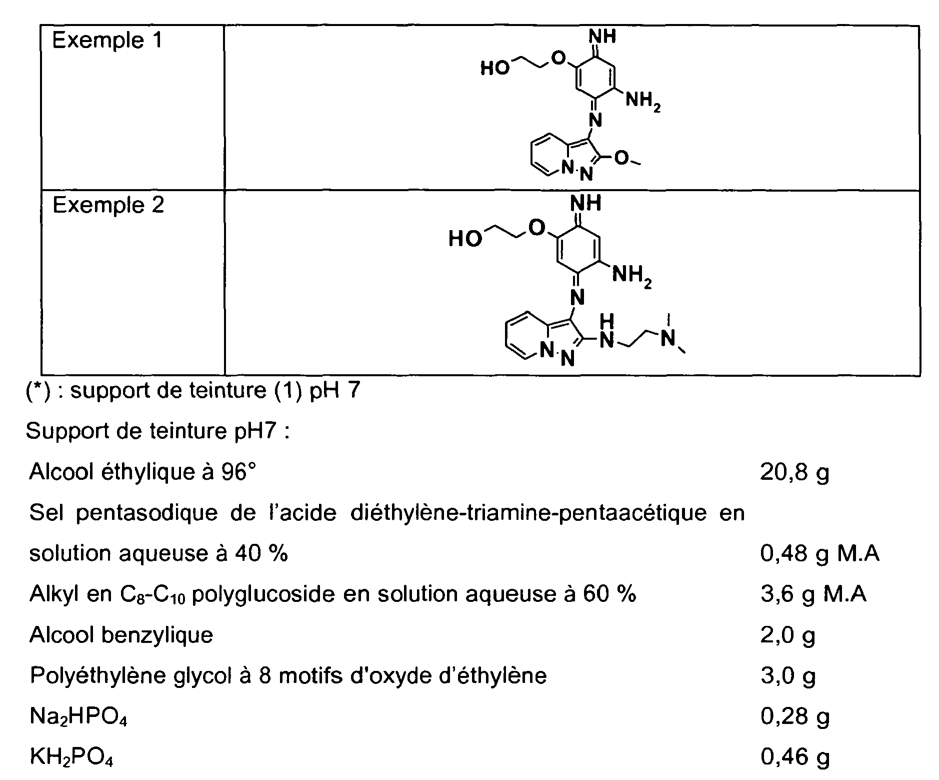

Chaque mélange obtenu est appliqué sur des mèches de cheveux gris à 90% de blancs. Après 30 minutes de pose, les mèches sont rincées, lavées avec un shampooing standard, rincées à nouveau puis séchées.

Les nuances obtenues figurent dans le tableau ci-dessous :

| Exemple | 1 | 2 |
|---|---|---|
| Nuance observée | Rouge intense | Bleu intense |

Pour les colorations en milieux oxydants : au moment de l'emploi, chacune des compositions décrites ci-dessus est mélangée avec un poids égal d'eau oxygénée à 20 volumes (6% en poids). On obtient un pH final de 7.

Les nuances obtenues figurent dans le tableau ci-dessous :

| Exemple | 1 | 2 |
|---|---|---|
| Nuance observée | rouge intense | Vert-Bleu intense |

### Exemples 3 et 4 de teinture en milieu basique

Les compositions tinctoriales suivantes sont préparées :

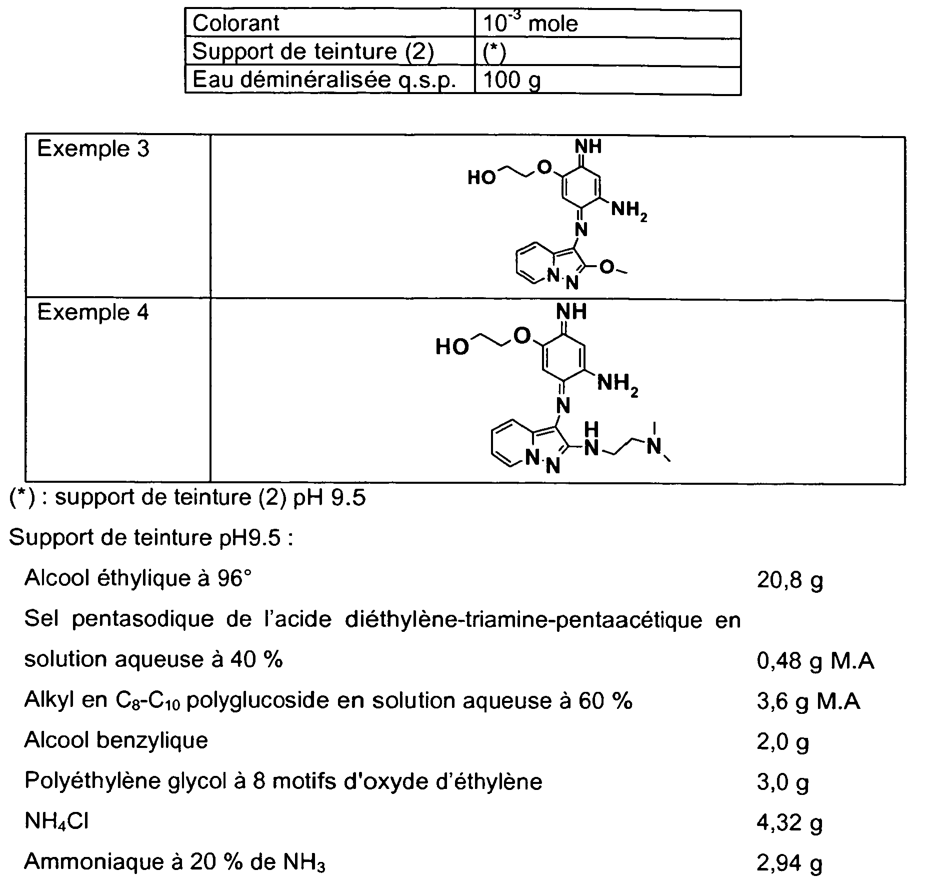

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 3 | 4 |
|---|---|---|
| Nuance observée | rouge intense | Vert-bleu |

Pour les milieux oxydants : au moment de l'emploi, chacune des compositions décrites ci-dessus est mélangée avec un poids égal d'eau oxygénée à 20 volumes (6% en poids). On obtient un pH final de 9,5.

Les nuances obtenues figurent dans le tableau ci-dessous :

| Exemple | 3 | 4 |
|---|---|---|
| Nuance observée | rouge intense | Vert-bleu |

## Revendications

1. Utilisation pour la coloration des fibres kératiniques d'au moins un composé choisi parmi les composés de type leuco de formule (I), les colorants de type azométhinique à motif pyrazolopyridine de formule (II) correspondant aux composés de formule (I), leurs formes mésomères, isomères et tautomères, ainsi que leurs sels d'addition avec un acide et leurs solvates : 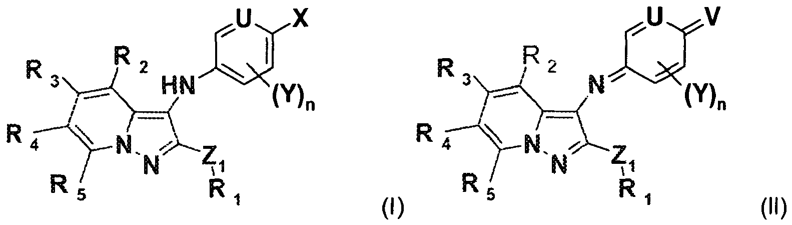 dans lesquelles :
• Z₁ représente un atome d'oxygène, un groupement NR₆, lorsque Z₁ représente NR₆ alors R₁ et R₆ peuvent former ensemble avec l'atome d'azote auquel ils sont rattachés un hétérocycle saturé, insaturé ou aromatique de 5 à 8 chaînons, éventuellement substitué,
Z₁ peut aussi représenter un radical divalent S, SO, SO₂ lorsque R₁ est un radical méthyle,
• R₁ et R₆ représentent indépendamment :
- un atome d'hydrogène,
- un radical alkyle en C₁-C₁₀, éventuellement substitué, le substituant pouvant éventuellement être un (hétéro)cycle saturé, insaturé ou aromatique de 5 à 8 chaînons, éventuellement substitué,
- un (hétéro)cycle saturé, insaturé ou aromatique de 5 à 8 chaînons, éventuellement substitué,
• R₂, R₃, R₄, R₅, indépendamment, représentent :
- un atome d'hydrogène,
- un radical alkyle en C₁-C₄ éventuellement substitué,
- un groupement choisi parmi NH₂, NHR₁₀, NR₁₁R₁₂, OH, OR₉, avec R₉ et R₁₀ représentant un alkyle en C₁-C₆ linéaire ou ramifié, éventuellement substitué, R₁₁ et R₁₂ identiques ou différents représentant un alkyle en C₁-C₆ linéaire ou ramifié, éventuellement substitué, R₁₁ et R₁₂ pouvant former ensemble avec l'atome d'azote auquel ils sont rattachés, un hétérocycle saturé, insaturé, ou aromatique, de 5 à 8 chaînons renfermant éventuellement un ou plusieurs autres hétéroatomes ou groupements choisis parmi N, O, S, SO₂, CO, l'hétérocycle étant éventuellement substitué,
• R₂, R₃, R₄, R₅, peuvent former deux à deux avec des radicaux adjacents un (hétéro)cycle saturé ou insaturé, éventuellement substitué,
• n est un entier compris entre 0 et 3 ;
• U représente CR ou N ;
• R représente
- un atome d'hydrogène,
- un radical alkyle en C₁-C₄ éventuellement substitué par un radical hydroxyle ;
- un radical alcoxy en C₁-C₄ éventuellement substitué par un radical hydroxyle ;
- un radical (di)alkyl(C₁-C₄)amino dont la partie alkyle est éventuellement substituée par un radical hydroxyle ;
• **X** représente :
- un radical hydroxyle;
- un groupement NR'₁R"₁ avec R'₁ et R"₁ choisis indépendamment parmi un atome d'hydrogène ; un radical alkyle en C₁-C₆ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy ;
lorsque R'₁ et R"₁ sont différents de l'hydrogène, R'₁ et R"₁ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, carboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
lorsque X représente un groupement NHR'₁ et que U représente un groupement CR dans lequel R désigne un radical alcoxy, alors X et U peuvent former un cycle à 6 chainons de type morpholine, éventuellement substitué par un ou plusieurs groupements alkyles en C₁-C₄ ;
• V représente
o un atome d'oxygène ;
o un groupement NR'₁ avec R'₁ choisi parmi un atome d'hydrogène ; un radical alkyle en C₁-C₆ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy ;
lorsque V représente un groupement NR'₁ et que U représente un groupement CR dans lequel R désigne un radical alcoxy, alors V et U peuvent former un cycle à 6 chainons de type morpholine, éventuellement substitué par un ou plusieurs groupements alkyles C₁-C₄ ;
• Y, identiques ou différents, représentent :
- un radical hydroxy ;
- un radical alkyle en C₁-C₄ ;
- un radical hydroxyalkyle C₁-C₄ ;
- un atome d'halogène tel qu'un atome de chlore, d'iode, de fluor ou de brome ;
- un atome d'oxygène substitué par un radical choisi parmi un radical alkyle en C₁-C₄, un radical aryle et un radical hétéroaryle, ceux-ci pouvant être substitués par un ou plusieurs radicaux hydroxy ;
- un groupement NR'₂R'₃ ;
R'₂ et R'₃, identiques ou différents, peuvent être choisis parmi
- un atome d'hydrogène ;
- un radical alkylcarbonyle en C₁-C₄ éventuellement substitué par un groupement ammonium quaternaire tel que par exemple un trialkylammonium ou par un hétérocycle azoté cationique ou non comme par exemple un groupement imidazole, un groupement thiazole, un groupement pyridine, un groupement pipéridine, un groupement pyrrolidine, un groupement pyrimidine, un groupement pyrazine, un groupement imidazolium, un groupement pyridinium, un groupement thiazolium, un groupement pyrrolidinium, un groupement piperidinium, un groupement pyrimidinium, ces hétérocycles azotés étant eux-mêmes éventuellement substitués par un ou plusieurs radicaux alkyle en C₁-C₄
- un radical aminocarbonyle ;
- un radical alkyle en C₁-C₆ éventuellement substitué par un ou plusieurs radicaux choisis parmi un radical hydroxy, (C₁-C₂)alcoxy, amino, (di)alkyl(C₁-C₂)amino ;
- un radical phényle éventuellement substitué par un ou plusieurs radicaux choisis parmi un radical hydroxy, amino, (C₁-C₂)alcoxy ;
R'₂ et R'₃ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
deux radicaux Y portés par deux atomes de carbone adjacents peuvent former ensemble avec les atomes de carbone auxquels ils sont rattachés un groupement cyclique ou hétérocyclique, saturé ou insaturé, aromatique ou non aromatique, comportant de 5 à 6 chaînons, par exemple un cycle benzène, pyrrole, pyrrolidine, pyrazole, furanne, pyrrolidine, morpholine ou imidazole, éventuellement substitué par un ou plusieurs radicaux alkyle en C₁-C₄.

2. Utilisation selon la revendication 1 dans laquelle Z₁ représente un atome d'oxygène, un radical NR₆ ou un radical NR₆ formant avec R₁ un hétérocycle.

3. Utilisation selon la revendication 2 dans laquelle le radical R₆ est choisi parmi un atome d'hydrogène, un radical alkyle en C₁-C₆ ou un radical alkyle en C₁-C₆ substitué par un radical hydroxy, un radical amino, un radical alkyl(C₁-C₄)amino, un radical dialkyl(C₁-C₄)amino, un radical alkyle en C₁-C₆ substitué par un hétérocycle azoté.

4. Utilisation selon l'une quelconque des revendications 1 à 3 dans laquelle le radical R₁ est choisi parmi un radical alkyle en C₁-C₆, un radical alkyle en C₁-C₆ substitué par un hydroxy, un radical alkyle en C₁-C₆ substitué par un amino ou (di)alkyl(C₁-C₄)amino, un radical alkyle en C₁-C₆ substitué par un hétérocycle azoté.

5. Utilisation selon l'une quelconque des revendications 1 à 4 dans laquelle R₂, R₃, R₄ et R₅ représentent indépendamment un atome d'hydrogène ou un radical alkyle en C₁-C₄ ou R₄ et R₅ forment ensemble un cycle de 5 à 8 chaînons.

6. Utilisation selon l'une quelconque des revendications 1 à 5 dans laquelle U représente CR ou N, et R représente un atome d'hydrogène, un radical alkyle en C₁ C₄, un radical alcoxy en C₁-C₄ éventuellement substitué par un radical hydroxyle, un radical (di)alkyl(C₁-C₄)amino dont la partie alkyle est éventuellement substituée par un radical hydroxyle.

7. Utilisation selon l'une quelconque des revendications 1 à 6 dans laquelle X représente un radical hydroxyle ; un groupement NR'₁R "₁ avec R'₁ et R"₁ choisis indépendamment parmi un atome d'hydrogène ; un radical alkyle en C₁-C₆ éventuellement substitué par un ou plusieurs radicaux hydroxyle ou R'1 et R"1 forment un hétérocycle.

8. Utilisation selon l'une quelconque des revendications 1 à 7 dans laquelle V représente un atome d'oxygène ; un groupement NR'₁ dans lequel R'₁ représente un atome d'hydrogène ou un radical alkyle en C₁-C₆ éventuellement substitué par un ou plusieurs radicaux hydroxyle.

9. Utilisation selon l'une quelconque des revendications 1 à 8 dans laquelle Y, identiques ou différents, représentent un radical hydroxy ; un radical alkyle en C₁-C₄ ; un atome d'halogène ; un atome d'oxygène substitué par un radical alkyle en C₁-C₄ pouvant être substitué par un ou plusieurs radicaux hydroxy ; un groupement NR'₂R'₃ ; R'₂ et R'₃, identiques ou différents, pouvant être choisis parmi un atome d'hydrogène ; un radical alkylcarbonyle en C₁-C₄ ; un radical aminocarbonyle ; un radical alkyle en C₁-C₆ éventuellement substitué par un ou plusieurs radicaux hydroxy ; R'₂ et R'₃ pouvant aussi former ensemble avec l'atome d'azote auquel ils sont rattachés un hétérocycle saturé comportant de 5 à 7 chaînons.

10. Utilisation selon l'une quelconque des revendications 1 à 9 dans laquelle le ou les composés sont choisis parmi les composés de type leuco de formule (I), les colorants de type azométhinique à motif pyrazolopyridine de formule (II) correspondant aux composés de formule (I), leurs formes mésomères, isomères, tautomères, ainsi que leurs sels d'addition avec un acide et leurs solvates.

11. Composition pour la coloration des fibres kératiniques comprenant, dans un milieu approprié pour la teinture, au moins un composé choisi parmi les composés de type leuco de formule (I) et les colorants de type azométhinique à motif pyrazolopyridine de formule (II) correspondant aux composés de formule (I) tels que définis à l'une quelconque des revendications 1 à 10, leurs formes mésomères isomères, et tautomères leurs sels d'addition avec un acide et leurs solvates, à l'exception du composé suivant : 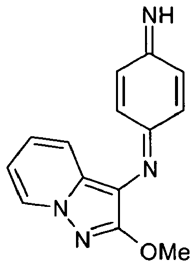 ainsi que ses formes mésomères et les formes isomères, tautomères, ses sels d'addition avec un acide et ses solvates.

12. Composition selon la revendication 11 comprenant au moins un agent oxydant.

13. Composé choisi parmi les composés de type leuco de formule (I) et les colorants de type azométhinique à motif pyrazolopyridine de formule (II) correspondant aux composés de formule (I) tels que définis à l'une quelconque des revendications 1 à 10, leurs formes mésomères, isomères et tautomères, leurs sels d'addition avec un acide et leurs solvates, à l'exception du composé suivant : 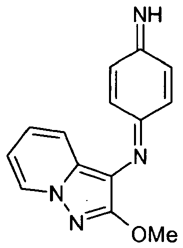 ainsi que ses formes mésomères, ses sels d'addition avec un acide et ses solvates.

14. Procédé de teinture des fibres kératiniques dans lequel on applique sur ces fibres au moins un composé choisi parmi les composés de type leuco de formule (I) et les colorants de type azométhinique à motif pyrazolopyridine de formule (II) correspondant aux composés de formule (I) tels que définis à l'une quelconque des revendications 1 à 10, leurs formes mésomères, les formes isomères et tautomères, leurs sels d'addition avec un acide et leurs solvates.

15. Dispositif à plusieurs compartiments, ou "kit" de teinture à plusieurs compartiments, dont un premier compartiment contient au moins un composé de formule (I) tel que défini à l'une quelconque des revendications 1 à 10 et un deuxième compartiment contient un agent oxydant, et éventuellement un composé de formule (II) tel que défini à l'une quelconque des revendications 1 à 10, un agent alcalin.

## Patentansprüche

1. Verwendung mindestens einer Verbindung, ausgewählt aus den Verbindungen vom Leuko-Typ der Formel (I), den Farbstoffen vom Azomethin-Typ mit einer Pyrazolopyridin-Einheit der Formel (II), die den Verbindungen der Formel (I) entsprechen, deren mesomeren, isomeren und tautomeren Formen, deren Säureadditionssalzen und deren Solvaten: 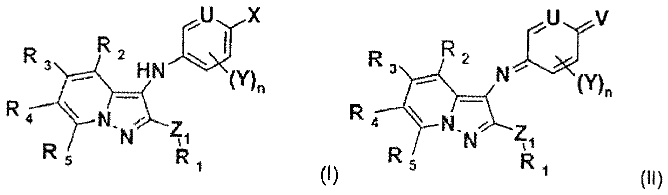 worin:
• Z₁ für ein Sauerstoffatom oder eine NR₆-Gruppe steht, dann, wenn Z₁ für NR₆ steht, R₁ und R₆ zusammen mit dem Stickstoffatom, an das sie gebunden sind, einen gegebenenfalls substituierten gesättigten, ungesättigten oder aromatischen 5-bis 8-gliedrigen Heterocyclus bilden,
Z₁ auch für einen zweiwertigen S-, SO- oder SO₂-Rest stehen kann, wenn R₁ für einen Methylrest steht;
• R₁ und R₆ unabhängig für:
- ein Wasserstoffatom;
- einen C₁-C₁₀-Alkylrest, der gegebenenfalls substituiert ist, wobei es sich bei dem Substituenten gegebenenfalls um einen gegebenenfalls substituierten gesättigten, ungesättigten oder aromatischen 5- bis 8-gliedrigen (Hetero)cyclus handeln kann,
- einen gegebenenfalls substituierten gesättigten, ungesättigten oder aromatischen 5- bis 8-gliedrigen (Hetero)cyclus
stehen,
• R₂, R₃, R₄ und R₅ unabhängig für:
- ein Wasserstoffatom;
- einen gegebenenfalls substituierten C₁-C₄-Alkylrest;
- eine aus NH₂, NHR₁₀, NR₁₁R₁₂, OH und OR₉ ausgewählte Gruppierung, wobei R₉ und R₁₀ für ein gegebenenfalls substituiertes lineares oder verzweigtes C₁-C₆-Alkyl stehen, R₁₁ und R₁₂ gleich oder verschieden sind und für ein gegebenenfalls substituiertes lineares oder verzweigtes C₁-C₆-Alkyl stehen, wobei R₁₁ und R₁₂ zusammen mit dem Stickstoffatom, an das sie gebunden sind, einen gesättigten, ungesättigten oder aromatischen 5- bis 8-gliedrigen Heterocyclus, der ein oder mehrere andere Heteroatome bzw. Gruppen, die aus N, O, S, SO₂ und CO ausgewählt sind, enthalten kann, bilden können, wobei der Heterocyclus gegebenenfalls substituiert ist,
stehen,
• R₂, R₃, R₄ und R₅ paarweise mit den benachbarten Resten einen gesättigten oder ungesättigten, gegebenenfalls substituierten (Hetero)cyclus bilden können,
• n für eine ganze Zahl zwischen 0 und 3 steht;
• U für CR oder N steht;
• R für
- ein Wasserstoffatom;
- einen C₁-C₄-Alkylrest, der gegebenenfalls durch einen Hydroxylrest substituiert ist;
- einen C₁-C₄-Alkoxyrest, der gegebenenfalls durch einen Hydroxylrest substituiert ist;
- einen (Di) (C₁-C₄)alkylaminorest, dessen Alkylteil gegebenenfalls durch einen Hydroxylrest substituiert ist;
steht;
• X für:
- einen Hydroxylrest;
- eine NR'₁R''₁-Gruppe, wobei R'₁ und R''₁ unabhängig voneinander aus einem Wasserstoff-atom; einem C₁-C₆-Alkylrest, der gegebenenfalls durch einen oder mehrere aus einem Hydroxy-, (C₁-C₂)Alkoxy-, Amino- und (Di) (C₁-C₂)alkylamino-rest ausgewählte Reste substituiert ist; und einem Phenylrest, der gegebenenfalls durch einen oder mehrere aus einem Hydroxy-, Amino-und (C₁-C₂)Alkoxyrest ausgewählte Reste substituiert ist; ausgewählt sind;
steht;
dann, wenn R'₁ und R''₁ von Wasserstoff verschieden sind, R'₁ und R''₁ zusammen mit dem Stickstoffatom, an das sie gebunden sind, einen gesättigten oder ungesättigten 5- bis 7-gliedrigen Heterocyclus, dessen Kohlenstoffatome durch ein Sauerstoff- oder Stickstoffatom ersetzt sein können, bilden können, wobei dieser Heterocyclus gegebenenfalls durch einen oder mehrere aus einem Halogenatom und einem Amino-, (Di)(C₁-C₄)alkylamino-, Hydroxy-, Carboxy-, Carboxamido-, (C₁-C₂)Alkoxy- und C₁-C₄-Alkylrest ausgewählte Reste, die gegebenenfalls durch einen oder mehrere Reste, die aus einem Hydroxy-, Amino-, (Di)alkylamino-, Alkoxy-, Carboxy- und Sulfonylrest ausgewählt sind, substituiert sein können, substituiert ist;
dann, wenn X für eine NHR'₁-Gruppe steht und U für eine CR-Gruppe, in der R für einen Alkoxyrest steht, steht, X und U einen 6-gliedrigen Ring vom Morpholin-Typ, der gegebenenfalls durch eine oder mehrere C₁-C₄-Alkylgruppen substituiert ist, bilden können;
• V für
o ein Sauerstoffatom;
o eine NR'₁-Gruppe, wobei R'₁ aus einem Wasserstoffatom; einem C₁-C₆-Alkylrest, der gegebenenfalls durch einen oder mehrere aus einem Hydroxy-, (C₁-C₂)Alkoxy-, Amino- und (Di)(C₁-C₂)alkylaminorest ausgewählte Reste substituiert ist; und einem Phenylrest, der gegebenenfalls durch einen oder mehrere aus einem Hydroxy-, Amino- und (C₁-C₂)Alkoxyrest ausgewählte Reste substituiert ist; ausgewählt ist;
steht;
dann, wenn V für eine NR'₁-Gruppe steht und U für eine CR-Gruppe, in der R für einen Alkoxyrest steht, steht, V und U einen 6-gliedrigen Ring vom Morpholin-Typ, der gegebenenfalls durch eine oder mehrere C₁-C₄-Alkylgruppen substituiert ist, bilden können;
• die Y-Reste gleich oder verschieden sind und für:
- einen Hydroxyrest;
- einen C₁-C₄-Alkylrest;
- einen C₁-C₄-Hydroxyalkylrest;
- ein Halogenatom wie ein Chlor-, Iod-, Fluoroder Bromatom;
- ein Sauerstoffatom, das durch einen Rest, der aus einem C₁-C₄-Alkylrest, einem Arylrest und einem Heteroarylrest ausgewählt ist, substituiert ist, wobei diese Reste durch einen oder mehrere Hydroxyreste substituiert sein können;
- eine NR'₂R'₃-Gruppe
stehen;
R'₂ und R'₃ gleich oder verschieden sind und aus:
- einem Wasserstoffatom;
- einem C₁-C₄-Alkylcarbonylrest, der gegebenenfalls durch eine quartäre Ammoniumgruppe wie beispielsweise ein Trialkylammonium oder durch einen kationischen oder nichtkationischen stickstoffhaltigen Heterocyclus wie beispielsweise eine Imidazolgruppe, eine Thiazolgruppe, eine Pyridingruppe, eine Piperidingruppe, eine Pyrrolidingruppe, eine Pyrimidingruppe, eine Pyrazingruppe, eine Imidazoliumgruppe, eine Pyridiniumgruppe, eine Thiazoliumgruppe, eine Pyrrolidinium-gruppe, eine Piperidiniumgruppe oder eine Pyrimidiniumgruppe substituiert ist, wobei diese stickstoffhaltigen Heterocyclen selbst gegebenenfalls durch einen oder mehrere C₁-C₄-Alkylreste substituiert sind;
- einem Aminocarbonylrest;
- einem C₁-C₆-Alkylrest, der gegebenenfalls durch einen oder mehrere aus einem Hydroxy-, (C₁-C₂)Alkoxy-, Amino- und (Di)(C₁-C₂)alkyl-aminorest ausgewählte Reste substituiert ist;
- einem Phenylrest, der gegebenenfalls durch einen oder mehrere aus einem Hydroxy-, Amino-und (C₁-C₂)Alkoxyrest ausgewählte Reste substituiert ist;
ausgewählt sein können;
R'₂ und R'₃ zusammen mit dem Stickstoffatom, an das sie gebunden sind, einen gesättigten oder ungesättigten 5- bis 7-gliedrigen Heterocyclus, dessen Kohlenstoffatome durch ein Sauerstoff- oder Stickstoffatom ersetzt sein können, bilden können, wobei dieser Heterocyclus gegebenenfalls durch einen oder mehrere aus einem Halogenatom und einem Amino-, (Di)(C₁-C₄)alkylamino-, Hydroxy-, Carboxy-, (Di)alkylcarboxamido-, (C₁-C₂)Alkoxy- und C₁-C₄-Alkylrest ausgewählte Reste, die gegebenenfalls durch einen oder mehrere Reste, die aus einem Hydroxy-, Amino-, (Di)alkylamino-, Alkoxy-, Carboxy- und Sulfonylrest ausgewählt sind, substituiert sein können, substituiert ist;
zwei Y-Reste an zwei benachbarten Kohlenstoff-atomen zusammen mit den Kohlenstoffatomen, an die sie gebunden sind, eine cyclische oder heterocyclische, gesättigte oder ungesättigte, aromatische oder nichtaromatische 5- bis 6-gliedrige Gruppe, beispielsweise einen Benzol-, Pyrrol-, Pyrrolidin-, Pyrazol-, Furan-, Pyrrolidin-, Morpholin- oder Imidazolring, der gegebenenfalls durch einen oder mehrere C₁-C₄-Alkylreste substituiert ist, bilden können;
zum Färben von Keratinfasern.

2. Verwendung nach Anspruch 1, wobei Z₁ für ein Sauerstoffatom, einen NR₆-Rest oder einen NR₆-Rest, der mit R₁ einen Heterocyclus bildet, steht.

3. Verwendung nach Anspruch 2, wobei der Rest R₆ aus einem Wasserstoffatom, einem C₁-C₆-Alkylrest oder einem C₁-C₆-Alkylrest, der durch einen Hydroxyrest, einen Aminorest, einen (C₁-C₄)Alkylaminorest oder einen Di(C₁-C₄)alkylaminorest substituiert ist, und einem C₁-C₆-Alkylrest, der durch einen stickstoffhaltigen Heterocyclus substituiert ist, ausgewählt ist.

4. Verwendung nach einem der Ansprüche 1 bis 3, wobei der Rest R₁ aus einem C₁-C₆-Alkylrest, einem C₁-C₆-Alkylrest, der durch ein Hydroxy substituiert ist, einem C₁-C₆-Alkylrest, der durch ein Amino oder (Di)(C₁-C₄)alkylamino substituiert ist, und einem C₁-C₆-Alkylrest, der durch einen stickstoffhaltigen Heterocyclus substituiert ist, ausgewählt ist.

5. Verwendung nach einem der Ansprüche 1 bis 4, wobei R₂, R₃, R₄ und R₅ unabhängig für ein Wasserstoffatom oder einen C₁-C₄-Alkylrest stehen oder R₄ und R₅ zusammen einen 5- bis 8-gliedrigen Ring bilden.

6. Verwendung nach einem der Ansprüche 1 bis 5, wobei U für CR oder N steht und R für ein Wasserstoff-atom, einen C₁-C₄-Alkylrest, einen C₁-C₄-Alkoxyrest, der gegebenenfalls durch einen Hydroxylrest substituiert ist, oder einen (Di) (C₁-C₄)alkylaminorest, dessen Alkylteil gegebenenfalls durch einen Hydroxylrest substituiert ist, steht.

7. Verwendung nach einem der Ansprüche 1 bis 6, wobei X für einen Hydroxylrest oder eine NR'₁R''₁-Gruppe steht, wobei R'₁ und R''₁ unabhängig voneinander aus einem Wasserstoffatom und einem C₁-C₆-Alkylrest, der gegebenenfalls durch einen oder mehrere Hydroxylreste substituiert ist, ausgewählt sind oder R'1 und R''₁ einen Heterocyclus bilden.

8. Verwendung nach einem der Ansprüche 1 bis 7, wobei V für ein Sauerstoffatom oder eine NR'₁-Gruppe steht, wobei R'₁ für ein Wasserstoffatom oder einen C₁-C₆-Alkylrest, der gegebenenfalls durch einen oder mehrere Hydroxylreste substituiert ist, steht.

9. Verwendung nach einem der Ansprüche 1 bis 8, wobei die Y-Reste gleich oder verschieden sind und für einen Hydroxyrest; einen C₁-C₄-Alkylrest; ein Halogenatom; ein Sauerstoffatom, das durch einen C₁-C₄-Alkylrest, der durch einen oder mehrere Hydroxyreste substituiert sein kann, substituiert ist; oder eine NR'₂R'₃-Gruppe stehen; R'₂ und R'₃ gleich oder verschieden sind und aus einem Wasserstoffatom; einem C₁-C₄-Alkylcarbonylrest; einem Aminocarbonylrest und einem C₁-C₆-Alkylrest, der gegebenenfalls durch einen oder mehrere Hydroxyreste substituiert ist; stehen; wobei R'₂ und R'₃ auch zusammen mit dem Stickstoffatom, an das sie gebunden sind, einen gesättigten 5- bis 7-gliedrigen Heterocyclus bilden können.

10. Verwendung nach einem der Ansprüche 1 bis 9, wobei die Verbindung bzw. die Verbindungen aus den Verbindungen vom Leuko-Typ der Formel (I), den Farbstoffen vom Azomethin-Typ mit einer Pyrazolopyridin-Einheit der Formel (II), die den Verbindungen der Formel (I) entsprechen, deren mesomeren, isomeren und tautomeren Formen, deren Säureadditionssalzen und deren Solvaten ausgewählt ist bzw. sind.

11. Zusammensetzung zum Färben von Keratinfasern, umfassend, in einem für die Färbung geeigneten Medium, mindestens eine Verbindung, die aus den Verbindungen vom Leuko-Typ der Formel (I) und den Farbstoffen vom Azomethin-Typ mit einer Pyrazolopyridin-Einheit der Formel (II), die den Verbindungen der Formel (I) entsprechen, gemäß der in einem der Ansprüche 1 bis 10 angegebenen Definition, deren mesomeren, isomeren und tautomeren Formen, deren Säureadditionssalzen und deren Solvaten ausgewählt ist, mit Ausnahme der folgenden Verbindung: 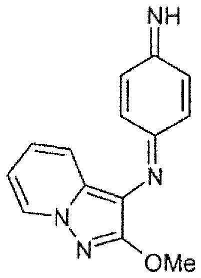 sowie deren mesomeren, isomeren und tautomeren Formen, deren Säureadditionssalzen und deren Solvaten.

12. Zusammensetzung nach Anspruch 11, die mindestens ein Oxidationsmittel umfasst.

13. Verbindung, ausgewählt aus den Verbindungen vom Leuko-Typ der Formel (I) und den Farbstoffen vom Azomethin-Typ mit einer Pyrazolopyridin-Einheit der Formel (II), die den Verbindungen der Formel (I) entsprechen, gemäß der in einem der Ansprüche 1 bis 10 angegebenen Definition, deren mesomeren, isomeren und tautomeren Formen, deren Säure-additionssalzen und deren Solvaten, mit Ausnahme der folgenden Verbindung: 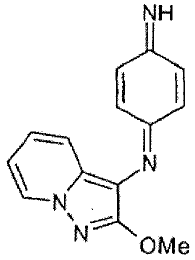 sowie deren mesomeren, isomeren und tautomeren Formen, deren Säureadditionssalzen und deren Solvaten.

14. Verfahren zum Färben von Keratinfasern, bei dem man auf diese Fasern mindestens eine eine Verbindung, die aus den Verbindungen vom Leuko-Typ der Formel (I) und den Farbstoffen vom Azomethin-Typ mit einer Pyrazolopyridin-Einheit der Formel (II), die den Verbindungen der Formel (I) entsprechen, gemäß der in einem der Ansprüche 1 bis 10 angegebenen Definition, deren mesomeren, isomeren und tautomeren Formen, deren Säure-additionssalzen und deren Solvaten ausgewählt ist, aufbringt.

15. Vorrichtung mit mehreren Kompartimenten oder Färbe-"Kit" mit mehreren Kompartimenten, wovon ein erstes Kompartiment mindestens eine Verbindung der Formel (I) gemäß der in einem der Ansprüche 1 bis 10 angegebenen Definition enthält und ein zweites Kompartiment ein Oxidationsmittel und gegebenenfalls eine Verbindung der Formel (II) gemäß der in einem der Ansprüche 1 bis 10 angegebenen Definition und ein alkalisches Mittel enthält.

## Claims

1. Use for dyeing keratin fibres of at least one compound chosen from the compounds of leuco form of formula (I), the dyes of azomethine type containing a pyrazolopyridine unit of formula (II) corresponding to the compounds of formula (I), the mesomeric forms, isomers and tautomers thereof, and also the acid-addition salts thereof and the solvates thereof: 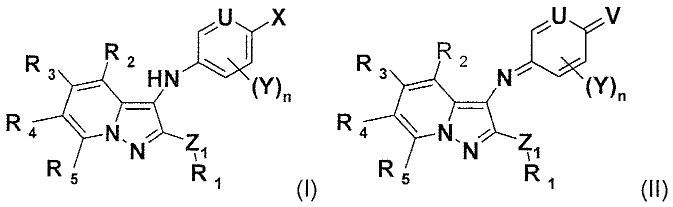 in which:
• Z₁ represents an oxygen atom, a group NR₆, when Z₁ represents NR₆ then R₁ and R₆ may form, together with the nitrogen atom to which they are attached, a saturated, unsaturated or aromatic, optionally substituted 5- to 8-membered heterocycle,
Z₁ may also represent a divalent radical S, SO or SO₂ when R₁ is a methyl radical,
• R₁ and R₆ independently represent:
- a hydrogen atom,
- an optionally substituted C₁-C₁₀ alkyl radical, the substituent possibly being a saturated, unsaturated or aromatic, optionally substituted 5- to 8-membered (hetero)cycle,
- a saturated, unsaturated or aromatic, optionally substituted 5- to 8-membered (hetero)cycle,
• R₂, R₃, R₄ and R₅ independently represent:
- a hydrogen atom,
- an optionally substituted C₁-C₄ alkyl radical,
- a group chosen from NH₂, NHR₁₀, NR₁₁R₁₂, OH and OR₉, with R₉ and R₁₀ representing a linear or branched, optionally substituted C₁-C₆ alkyl, R₁₁ and R₁₂, which may be identical or different, representing an optionally substituted linear or branched C₁-C₆ alkyl, R₁₁ and R₁₂ possibly forming, together with the nitrogen atom to which they are attached, a saturated, unsaturated or aromatic 5-to 8-membered heterocycle optionally containing one or more other heteroatoms or groups chosen from N, O, S, SO₂ and CO, the heterocycle being optionally substituted,
• R₂, R₃, R₄ and R₅ may form in pairs, with adjacent radicals, a saturated or unsaturated, optionally substituted (hetero)cycle,
• n is an integer between 0 and 3;
• U represents CR or N;
• R represents
- a hydrogen atom,
- a C₁-C₄ alkyl radical optionally substituted with a hydroxyl radical;
- a C₁-C₄ alkoxy radical optionally substituted with a hydroxyl radical;
- a (di)(C₁-C₄)alkylamino radical in which the alkyl part is optionally substituted with a hydroxyl radical;
• X represents:
- a hydroxyl radical;
- a group NR'₁R''₁ with R'₁ and R''₁ chosen independently from a hydrogen atom; a C₁-C₆ alkyl radical optionally substituted with one or more radicals chosen from a hydroxyl radical, (C₁-C₂)alkoxy, amino, (di)(C₁-C₂)alkylamino; a phenyl radical optionally substituted with one or more radicals chosen from hydroxyl, amino and (C₁-C₂)alkoxy radicals;
when R'₁ and R''₁ are other than a hydrogen, R'₁ and R''₁ may form, together with the nitrogen atom to which they are attached, a saturated or unsaturated 5-to 7-membered heterocycle, the carbon atoms of which may be replaced with an oxygen or nitrogen atom, this heterocycle being optionally substituted with one or more radicals chosen from a halogen atom and an amino, (di)(C₁-C₄)alkylamino, hydroxyl, carboxyl, carboxamido, (C₁-C₂)alkoxy or C₁-C₄ alkyl radical, optionally substituted with one or more radicals chosen from hydroxyl, amino, (di)alkylamino, alkoxy, carboxyl and sulfonyl radicals;
when X represents a group NHR'₁ and when U represents a group CR in which R denotes an alkoxy radical, then X and U may form a 6-membered ring of morpholine type, optionally substituted with one or more C₁-C₄ alkyl groups;
• V represents
o an oxygen atom;
o a group NR'₁ with R'₁ chosen from a hydrogen atom; a C₁-C₆ alkyl radical optionally substituted with one or more radicals chosen from hydroxyl, (C₁-C₂)alkoxy, amino and (di)(C₁-C₂)alkylamino radicals; a phenyl radical optionally substituted with one or more radicals chosen from hydroxyl, amino and (C₁-C₂)alkoxy radicals;
when V represents a group NR'₁ and when U represents a group CR in which R denotes an alkoxy radical, then V and U may form a 6-membered ring of morpholine type, optionally substituted with one or more C₁-C₄ alkyl groups;
• Y, which may be identical or different, represent:
- a hydroxyl radical;
- a C₁-C₄ alkyl radical;
- a C₁-C₄ hydroxyalkyl radical;
- a halogen atom such as a chlorine, iodine, fluorine or bromine atom;
- an oxygen atom substituted with a radical chosen from a C₁-C₄ alkyl radical, an aryl radical and a heteroaryl radical, these radicals possibly being substituted with one or more hydroxyl radicals;
- a group NR'₂R'₃;
R'₂ and R'₃, which may be identical or different, may be chosen from
- a hydrogen atom;
- a C₁-C₄ alkylcarbonyl radical optionally substituted with a quaternary ammonium group, for instance a trialkylammonium or with a cationic or non-cationic nitrogenous heterocycle, for instance an imidazole group, a thiazole group, a pyridine group, a piperidine group, a pyrrolidine group, a pyrimidine group, a pyrazine group, an imidazolium group, a pyridinium group, a thiazolium group, a pyrrolidinium group, a piperidinium group and a pyrimidinium group, these nitrogenous heterocycles themselves being optionally substituted with one or more C₁-C₄ alkyl radicals;
- an aminocarbonyl radical;
- a C₁-C₆ alkyl radical optionally substituted with one or more radicals chosen from hydroxyl, (C₁-C₂)alkoxy, amino and (di)(C₁-C₂)alkylamino radicals;
- a phenyl radical optionally substituted with one or more radicals chosen from hydroxyl, amino and (C₁-C₂)alkoxy radicals;
R'₂ and R'₃ may form, together with the nitrogen atom to which they are attached, a saturated or unsaturated, 5- to 7-membered heterocycle, the carbon atoms of which may be replaced with an oxygen or nitrogen atom, this heterocycle being optionally substituted with one or more radicals chosen from a halogen atom and amino, (di)(C₁-C₄)alkylamino, hydroxyl, carboxyl, (di)alkylcarboxamido, (C₁-C₂)alkoxy and C₁-C₄ radicals, optionally substituted with one or more radicals chosen from hydroxyl, amino, (di)alkylamino, alkoxy, carboxyl and sulfonyl radicals;
two radicals Y borne by two adjacent carbon atoms may form, together with the carbon atoms to which they are attached, a saturated or unsaturated, aromatic or non-aromatic, 5- to 6-membered cyclic or heterocyclic group, for example a benzene, pyrrole, pyrrolidine, pyrazole, furan, pyrrolidine, morpholine or imidazole ring, optionally substituted with one or more C₁-C₄ alkyl radicals.

2. Use according to Claim 1, in which Z₁ represents an oxygen atom, a radical NR₆ or a radical NR₆ forming with R₁ a heterocycle.

3. Use according to Claim 2, in which the radical R₆ is chosen from a hydrogen atom, a C₁-C₆ alkyl radical or a C₁-C₆ alkyl radical substituted with a hydroxyl radical, an amino radical, a (C₁-C₄)alkylamino radical, a di (C₁-C₄) alkylamino radical and a C₁-C₆ alkyl radical substituted with a nitrogenous heterocycle.

4. Use according to any one of Claims 1 to 3, in which the radical R₁ is chosen from a C₁-C₆ alkyl radical, a C₁-C₆ alkyl radical substituted with a hydroxyl radical, a C₁-C₆ alkyl radical substituted with an amino or (C₁-C₄) alkylamino radical, and a C₁-C₆ alkyl radical substituted with a nitrogenous heterocycle.

5. Use according to any one of Claims 1 to 4, in which R₂, R₃, R₄ and R₅ independently represent a hydrogen atom or a C₁-C₄ alkyl radical, or R₄ and R₅ together form a 5- to 8-membered ring.

6. Use according to any one of Claims 1 to 5, in which U represents CR or N, and R represents a hydrogen atom, a C₁-C₄ alkyl radical, a C₁-C₄ alkoxy radical optionally substituted with a hydroxyl radical, or a (di)(C₁-C₄)alkylamino radical in which the alkyl part is optionally substituted with a hydroxyl radical.

7. Use according to any one of Claims 1 to 6, in which X represents a hydroxyl radical; a group NR'₁R"₁ with R'₁ and R"₁ independently chosen from a hydrogen atom; a C₁-C₆ alkyl radical optionally substituted with one or more hydroxyl radicals, or R'₁ and R"₁ form a heterocycle.

8. Use according to any one of Claims 1 to 7, in which V represents an oxygen atom; a group NR'₁ in which R'₁ represents a hydrogen atom or a C₁-C₆ alkyl radical optionally substituted with one or more hydroxyl radicals.

9. Use according to any one of Claims 1 to 8, in which Y, which may be identical or different, represent a hydroxyl radical; a C₁-C₄ alkyl radical; a halogen atom; an oxygen atom substituted with a C₁-C₄ alkyl radical, which may be substituted with one or more hydroxyl radicals; a group NR'₂R'₃; R'₂ and R'₃, which may be identical or different, possibly being chosen from a hydrogen atom; a C₁-C₄ alkylcarbonyl radical; an aminocarbonyl radical; a C₁-C₆ alkyl radical optionally substituted with one or more hydroxyl radicals; R'₂ and R'₃ also possibly forming, together with the nitrogen atom to which they are attached, a saturated 5- to 7-membered heterocycle.

10. Use according to any one of Claims 1 to 9, in which the compound(s) are chosen from the compounds of leuco type of formula (I), the dyes of azomethine type containing a pyrazolopyridine unit of formula (II) corresponding to the compounds of formula (I), the mesomeric forms, isomers and tautomers thereof, and also the acid-addition salts thereof and the solvates thereof.

11. Composition for dyeing keratin fibres, comprising, in a suitable dyeing medium, at least one compound chosen from the compounds of leuco type of formula (I) and the dyes of azomethine type containing a pyrazolopyridine unit of formula (II) corresponding to the compounds of formula (I) as defined in any one of Claims 1 to 10, the mesomeric forms, isomers and tautomers thereof, the acid-addition salts thereof and the solvates thereof, with the exception of the following compound: 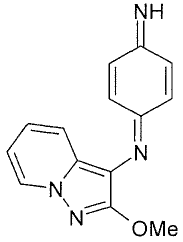 and also the mesomeric forms thereof and the isomers and tautomers, the acid-addition salts thereof and the solvates thereof.

12. Composition according to Claim 11, comprising at least one oxidizing agent.

13. Compound chosen from the compounds of leuco type of formula (I) and the dyes of azomethine type containing a pyrazolopyridine unit of formula (II) corresponding to the compounds of formula (I) as defined in any one of Claims 1 to 10, the mesomeric forms, isomers and tautomers thereof, the acid-addition salts thereof and the solvates thereof, with the exception of the following compound: 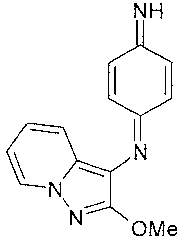 and also the mesomeric forms thereof, the acid-addition salts thereof and the solvates thereof.

14. Process for dyeing keratin fibres, in which at least one compound chosen from the compounds of leuco type of formula (I) and the dyes of azomethine type containing a pyrazolopyridine unit of formula (II) corresponding to the compounds of formula (I) as defined in any one of Claims 1 to 10, the mesomeric forms, isomers and tautomers thereof, the acid-addition salts thereof and the solvates thereof, is applied to these fibres.

15. Multi-compartment device, or multi-compartment dyeing "kit", a first compartment of which contains at least one compound of formula (I) as defined in any one of Claims 1 to 10 and a second compartment contains an oxidizing agent and optionally a compound of formula (II) as defined in any one of Claims 1 to 10, an alkaline agent.
